# EUROPEAN PATENT SPECIFICATION

(11) **EP 1 455 579 B1**
(45) Date of publication and mention of the grant of the patent: **06.09.2006**
(21) Application number: 02785837.2
(22) Date of filing: 05.12.2002
(51) Int. Cl.: A01N 43/54, C07D 403/04

(54) **PYRAZOLES-PYRIMIDINE FUNGICIDES**
PYRAZOLPYRIMIDINFUNGIZIDE
FONGICIDES A BASE DE PYRAZOLES-PYRIMIDINE

(30) Priority: 10.12.2001 GB 0129476
(43) Date of publication of application: 15.09.2004
(73) Proprietor: Syngenta Participations AG, 4058 Basel (CH)
(72) Inventor: MUELLER, Urs, Syngenta Crop Protection AG, CH-4058 Basel (CH); EBERLE, Martin,, 4103 Bottmingen (CH); PILLONEL, Christian, Syngenta Crop Protection AG, CH-4058 Basel (CH)
(74) Representative: Waterman, John Richard
(86) International application number: PCT/IB2002/005146
(87) International publication number: WO 2003/049542

(56) References cited:
- WO-A-01/12621
- WO-A-01/60816
- WO-A-01/93682
- WO-A-02/092573

## Description

The present invention relates to novel N-[4-(pyrazol-4-yl)-pyrimidin-2-yl]-N-phenyl-amine derivatives, to a method of protecting plants against attack or infestation by phytopathogenic organisms, such as nematodes or insects or especially microorganisms, preferably fungi, bacteria and viruses, or combinations of two or more of these organisms, by applying an N-[4-(pyrazol-4-yl)-pyrimidin-2-yl]-N-phenyl-amine derivative as specified hereinafter to a part and/or to the site of a plant, to the use of said derivative for protecting plants against said organisms, and to compositions comprising said derivative as the active component. The invention further relates to the preparation of these novel N-[4-(pyrazol-4-yl)-pyrimidin-2-yl]-N-phenyl-amine derivatives.

Certain azolyl-pyrirnidin-2-yl-amine derivatives and their pharmaceutical use have been described in WO 01/12621; pyrimidine kinase inhibitors are disclosed in WO01/60816 and WO02/092573 and pyridyl-pyrimidines are disclosed as plant fungicides in WO0 1/93682.

Surprisingly, it has now been found that the new N-[4-(pyrazol-4-yl)-pyrimidin-2-yl]-N-phenyl-amine derivatives according to the present invention are effective in plant protection showing advantageous properties in the treatment of plant diseases caused by phytopathogenic microorganisms. Further the fungicidal activity allows the compounds according to present invention to be employed in controlling fungi in related areas, e.g. in protection of technical materials, including wood and wood related technical products, in food storage, in hygiene management, etc.

The novel N-[4-(pyrazol-4-yl)-pyrimidin-2-yl]-N-phenyl-amine derivatives according to the invention are those of the formula I 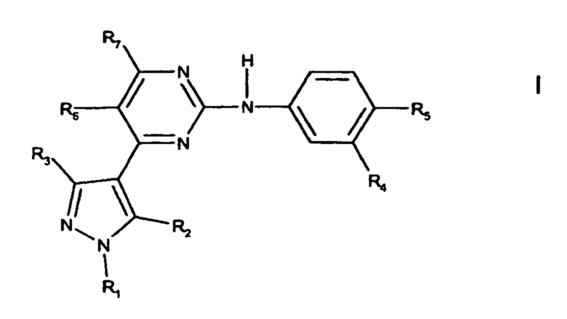 wherein
R₁ is hydrogen, COCH₃, COCH₂CH₃, SO₂N(CH₃)₂, COOCH₃, COOCH₂CH₃, CON(CH₃)₂, SO₂CH₃, CH₂C≡CH, methyl, ethyl, n-propyl or n-butyl;
R₂ is hydrogen or amino;
R₃ is phenyl or thienyl either of which may be optionally substituted by one, two or three substituents R₁₂ wherein each of the Regroups may be the same or different;
R₄ is hydrogen, hydroxy, cyano, fluorine, chlorine, bromine, 2-hydroximino-ethyl, 2-methoximino-ethyl, C₁-C₄alkyl, C₁-C₄alkoxy, C₁-C₄haloalkoxy, C₁-C₄haloalkyl, C₁-C₄cyanoalkyl, C₁-C₄alkanoyloxy, C₁-C₄hydroxyalkyl, C₁-C₄haloalkanoyloxy, C₁-C₄alkanoyl-C₁-C₆aminoalkyl, C₁-C₄alkanoyloxy-C₁-C₄alkyl, C₁-C₄alkanoyl, C₁-C₄alkylthio, C₁-C₄alkoxycarbonyl, heteroaryl or heterocyclyl, C₁-C₄alkanoyloxy, C₁-C₄alkyloxycarbonyloxy, C₁₋C₄alkanoyloxy, di(C₁-C₄alkyl)aminoC₁-C₄alkanoyloxy or di(C₁-C₄alkyl)aminosulfonyloxy,
R₅ is hydrogen, C₁-C₃ alkyl, hydroxy, C₁-C₃ alkoxy or halogen;
R₆ is hydrogen, C₁-C₃ alkyl, halogen or C₁-C₃ alkoxy,
R₇ is hydrogen, C₁-C₃ alkyl, halogen or C₁-C₃ alkoxy,
R₁₂ is halogen, C₁-C₄ alkyl or C₁-C₄ alkoxy.

The definition symbols and generic expressions used above are defined as below. Where radicals are combined from other sub-radicals, the definition of resulting new radical likewise follows from the combination of the definitions of said sub-radicals.

In this document "halogen" or the prefix "halo" shall include fluorine, chlorine, bromine and iodine. Preferably, this definition designates substitution with fluorine or chlorine.

Alkyl- as a group *per se* and as a structural element of hydroxyallcyl, aminoalkyl, cyanoalkyl, dialkylamino, cycloalkylalkyl, alkoxy, alkoxyalkyl, alkylthio, alkylsulfinyl, alkylsulfonyl, alkenyl, alkynyl, alkenylyoxy, alkynyloxy, alkylene, alkanoyl, alkanoyloxy, alkanoylamino, arylalkyl, heteroarylalkyl, haloalkyl or haloalkoxy - is preferably C₁-C₆alkyl, more preferably C₁-C₄alkyl. The alkyl, alkenyl and alkynyl radicals may be straight-chain or branched. This applies also to the alkyl, alkenyl or alkynyl parts of other alkyl-, alkenyl- or alkynyl-containing groups.

Depending upon the number of carbon atoms mentioned, alkyl on its own or as part of another substituent is to be understood as being, for example, methyl, ethyl, propyl, butyl, pentyl, hexyl, heptyl, octyl and the isomers thereof, for example isopropyl, isobutyl, tert-butyl or sec-butyl, isopentyl or tert-pentyl.

Lower alkyl [as a group *per se* and as a structural element of lower (hydroxyalkyl, aminoalkyl, cyanoalkyl, dialkylamino, cycloalkylalkyl, alkoxy, alkoxyalkyl, alkylthio, alkylsulfinyl, alkylsulfonyl, alkenyl, alkynyl, alkenylyoxy, alkynyloxy, alkylene, alkanoyl, alkanoyloxy, alkanoylamino, arylalkyl, heteroarylalkyl, haloalkyl or haloalkoxy)] is C1-3 alkyl and in particular methyl or ethyl are preferred.

Optionally substituted alkyl groups carry one to four, preferably not more than two further substituents selected from the group halogen, C₁-C₄alkoxy, C₁-C₄alkylthio, phenyl, halophenyl, C₁-C₄alkylphenyl, benzyl, halobenzyl and C₁-C₄alkylbenzyl.

Alkoxy includes methoxy, ethoxy, propyloxy, isopropyloxy, butoxy, isobutyloxy, sec-butyloxy, tert-butyloxy, and the diverse pentyloxy and hexyloxy.

Depending upon the number of carbon atoms mentioned, alkenyl as a group or as a structural element of other groups is to be understood as being, for example, ethenyl, allyl, 1-propen-1-yl, 2-propen-1-yl, 1-propen-3-yl, 1-buten-2-yl, 1-buten-3-yl, 1-penten-1-yl, 2-penten-1-yl, 1-penten-3-yl, hexen-1-yl, 4-methyl-3-pentenyl or 4-methyl-3-hexenyl. Optionally substituted alkenyl groups carry one to four, preferably not more than two further substituents selected from the group halogen, C₁-C₄alkoxy, C₁-C₄alkylthio, phenyl, halophenyl, C₁-C₄alkylphenyl, benzyl, halobenzyl and C₁-C₄alkylbenzyl. Examples are stryryl, chloroallyl, dichloroallyl, trichlorovinyl, 4,4,4-trifluoro-2-butenyl.

Alkynyl as a group or as a structural element of other groups is for example, ethynyl, propyn-1-yl, propyn-2-yl, butyn-1-yl, butyn-2-yl, 1-methyl-2-butynyl, hexyn-1-yl, 1-ethyl-2-butynyl or octyn-1-yl. Optionally substituted alkynyl groups carry one to four, preferably not more than two further substituents selected from the group halogen, C₁-C₄alkoxy, C₁-C₄alkylthio, phenyl, halophenyl, C₁-C₄alkylphenyl, amino, C₁-C₄alkylamino, di-(C₁-C₄alkyl)-amino, benzyl, halobenzyl and C₁-C₄alkylbenzyl. Examples are phenylethinyl, phenylpropargyl, chloropropargyl or 5,5,5-trifluoro-2-pentinyl. Alkenyloxy and alkynyloxy have analogous designations, employing the exemplified alkenyl and alkynyl radicals

Alkylene designates a bivalent bridge member, being linked at both ends of the hydrocarbon chain, which may be straight or branched. Typical examples are methylene, 1,2-ethylene, 1-methyl-1,2-ethylene, 1,2-dimethyl-1,2-ethylene, 1,1-dimethyl-1,2-ethylene, 1-ethyl-1,2-ethylene, 1-propyl-1,2-ethylene, 1-butyl-1,2-ethylene, 1,3-propylene, etc.. Where alkylene is attached to two oxygen atoms at both ends, and both oxygen atoms are in turn bound to the same carbon atom as in the definition of R₄, then the functional group is that of a ketale.

Cycloalkyl is, depending upon the number of carbon atoms mentioned, cyclopropyl, cyclobutyl, cyclopentyl, cyclohexyl, bicyclopentyl, bicyclohexyl or cycloheptyl. Likewise, cycloalkyl-alkyl or alkyl-cycloalkyl-alkyl encompass cyclopropylmethyl, cyclopropylethyl, cyclopentylmethyl, cyclohexylmethyl, cyclopentylethyl, cyclohexylethyl, methyl-cyclopropylmethyl, methyl-cyclopropylethyl, dimethyl-cyclopropylmethyl, dimethyl-cyclopropylethyl, methyl-cyclopentylmethyl, methyl-cyclohexylmethyl, methyl-cyclopentylethyl, methyl-cyclohexylethyl, ethyl-cyclopentylmethyl, ethyl-cyclohexylinethyl, ethyl-cyclopentylethyl, ethyl-cyclohexylethyl, dimethyl-cyclopentylmethyl, dimethyl-cyclohexylmethyl, dimethyl-cyclopentylethyl or dimethyl-cyclohexylethyl, etc..

Hydroxyalkyl preferably encompasses hydroxymethyl, 1-hydroxyethyl, 1-hydroxypropyl, 2-hydroxyethyl, 2-hydroxypropyl, 2-hydroxy-2-methyl-ethyl, 3-hydroxypropyl and various hydroxybutyl radicals.

Alkoxyalkyl is meant to include without limiting the definition thereof:
methoxymethyl, ethoxymethyl, propyloxymethyl, isopropyloxymethyl, butyloxymethyl, 2-methoxyethyl, 2-ethoxyethyl, 2-propyloxyethyl, 2-isopropyloxyethyl, 2-butyloxyethyl, 1-methoxyethyl, 1-ethoxyethyl, 1-propyloxyethyl, 1-isopropyloxyethyl, 1-butyloxyethyl, 1-methoxypropyl, 1-ethoxypropyl, 1-propyloxypropyl, 1-isopropyloxypropyl, 1-butyloxypropyl, 2-methoxypropyl, 2-ethoxypropyl, 2-propyloxypropyl, 2-isopropyloxypropyl, 2-butyloxypropyl, 3-methoxypropyl, 3-ethoxypropyl, 3-propyloxypropyl, 3-isopropyloxypropyl, 3-butyloxypropyl, methoxybutyl, ethoxybutyl, propyloxybutyl, and butyloxybutyl.

Alkoxycarbonyl is for example: methoxycarbonyl, ethoxycarbonyl, propyloxycarbonyl, isopropyloxycarbonyl, butyloxycarbonyl, s-butyloxycarbonyl, or terbutyloxycarbonyl.

Within this document alkanoyl includes the aliphatic acyl radicals, e.g. formyl, acetyl, propionyl, isopropionyl, butyryl, isobutyryl, sec-butyryl, tert-butyryl, valeryl, isovaleryl, sec-valeryl and pivaloyl. These radicals are frequently employed in the alkanoyloxyalkyl and alkanoylaminoalkyl groups as defined for this invention.

Aminoalkyl designates aminomethyl, aminoethyl, aminopropyl, aminobutyl and the isomeric forms thereof. Likewise alkylaminoalkyl and dialkylaminoalkyl include for example methylaminomethyl, ethylaminomethyl, propylaminomethyl, methylaminoethyl, ethylaminoethyl, propylaminoethyl, methylaminopropyl, ethylaminopropyl, dimethylaminomethyl, dimethylaminoethyl, dimethylaminopropyl. ethyl-methylaminomethyl, ethyl-methylaminoethyl, ethyl-methylaminomethyl, ethyl-methylaminopropyl, etc..

Haloalkyl stands for halogenated alkyl, preferably based on C₁-C₆haloalkyl, more preferably on C₁-C₄alkyl, which is linear or branched, and is substituted by one or more, for example in case of substitution with halogen atoms all hydrogens of the basic hydrocarbon structure may be replaced by halogen atoms, being all identical or different. Typical examples are CHCl₂, CH₂F, CCl₃, CH₂Cl, CHF₂; CF₃, CH₂CH₂Br, C₂Cl₅, CH₂Br, CHClBr, CF₃CH₂, C₂F₅, C₃F₇, C₄F₉, C₅F₁₁, CH₂CH₂Cl, CH(CH₃)-CH₂-CH₂Cl, CH₂CH₂F, CH(CH₃)-CH₂-CH₂F, C(CH₃)₂-CH₂-Cl, C(CH₃)₂-CH₂-CF₃, C(CH₃)-CH₂-CH₂-CF₃, etc..

Haloalkoxy stands for halogenated alkoxy which preferably based on C₁-C₆alkoxy, more preferably on C₁-C₄alkoxy, which is linear or branched, and is substituted by one or more, for example in the case of halo-ethyl up to five halogen atoms. Especially fluorine is preferred as a halogen substituent in alkoxy groups, including trifluoromethoxy and 1,1,2,2-tetrafluoroethoxy as prominent members.

Cyanoalkyl is meant to include without limiting the definition thereof: cyanomethyl, 1-cyanoethyl, 2-cyanoethyl, 1-cyanopropyl, 2-cyanopropyl, 3-cyanopropyl, 1-cyanoisopropyl, 2-cyanoisopropyl, the isomeric cyanobutyl, cyanopentyl and cyanohexyl. Cyanomethyl and cyanoethyl are preferred.

Aryl typically includes aromatic hydrocarbon rings like phenyl, naphthyl, anthracenyl, phenanthrenyl and biphenyl like 1,3-biphenyl and 1,4-biphenyl, with phenyl being preferred. The same definition applies where aryl is part of arylalkyl (also known as aralkyl). The most prominent examples for arylalkyl are benzyl and phenethyl.

Heteroaryl stands for aromatic ring systems comprising mono-, bi- or tricyclic systems wherein at least one oxygen, nitrogen or sulfur atom is present as a ring member. The same definition applies where heteroaryl is part of heteroarylalkyl. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolinyl, isoquinolinyl, phthalazinyl, quinoxalinyl, quinazolinyl, cinnolinyl and naphthyridinyl. The heteroaryl group may be bond to the basic molecular structure of formula I via a carbon or a nitrogen atom.

Heterocyclyl designates fully or partially hydrogenated heteroaryl ring system as outlined above. Typical examples include 2-tetrahydrofuranyl, 3-tetrahydrofuranyl, 5-hydroxy-tetrathydrofuran-2-yl, 2-isoxazolinyl, 3-isoxazolinyl, 4- isoxazolinyl, 5-isoxazolinyl, pyrrolidin-2-on-5-yl, N-pyrrolidinyl, 2-pyrrolidinyl, 2-pyrrolin-5-yl, etc.

Where the above aryl, heterocyclyl and heteroaryl groups may be optionally substituted, this means that they may carry one or more identical or different substituents. Normally not more than three substituents are present at the same time. Examples of substituents of aryl, heterocyclyl or heteroaryl groups are: amino, C₁-C₄alkylamino, di-C₁₋C₄alkylamino, halogen, hydroxy, mercapto, cyano, C₁-C₆alkyl, C₁-C₆alkoxy, C₁-C₆alkylthio, C₁-C₆alkylsulfinyl, C₁-C₆alkylsulfonyl, C₂-C₆al-kenyloxy and C₂-C₆alkynyloxy.

Typical examples for substituted aryl include 4-chlorophenyl, 4-bromophenyl, 3,4-dichlorophenyl, 4-chloro-3-fluorophenyl, 3-chloro-4-fluorophenyl, 4-methylphenyl, 4-ethylphenyl, 4-propargyloxyphenyl, 4-allyloxyphenyl, 3,4-dioxomethylenyl-phenyl, 3,4-dioxoethylenyl-phenyl, 3-methylphenyl, 4-fluorophenyl, 4-ethenylphenyl, 4-ethynylphenyl, 4-propylphenyl, 4-isopropylphenyl, 4-tert.butylphenyl, 4-ethoxyphenyl, 4-ethynyloxyphenyl, 4-methylmercaptophenyl, 4-methylsulfonylphenyl, 4-cyanophenyl, 4-methoxycarbonyl-phenyl, 3-bromophenyl, 3-chlorophenyl, 2-chlorophenyl, 2,4-dichlorophenyl, 3,4,5-trichlorophenyl, 3,4-difluorophenyl, 3,4-dibromophenyl, 3,4-dimethoxyphenyl, 3,4-dimethylphenyl, 3-chloro-4-cyanophenyl, 4-chloro-3-cyanophenyl, 3-bromo-4-methylphenyl, 4-methoxy-3-methylphenyl, 3-fluoro-4-methoxyphenyl, 4-chloro-3-methylphenyl, 4-chloro-3-trifluoromethyl-phenyl, 4-bromo-3-chlorophenyl, 4-trifluoromethylphenyl, 4-trifluoromethoxyphenyl, 4-methoxyphenyl, etc.

Typical examples for substituted heteroaryl include 5-methyl-2-thienyl, 5-chloro-2-thienyl, 3-chloro-2-thienyl, 5-methyl-2-furyl, 4-methyl-oxazol-5-yl, 5-hydroxy-pyrazol-I-yl, 5-hydroxy-3-methyl-pyrazol-1-yl, 5-amino-3-methyl-pyrazol-1-yl, 5-amino-pyrazol-1-yl, 5-methyl-[1,3,4]-oxadiazol-2-yl, 5-ethyl-[1,3,4]-oxadiazol-2-yl, 2-amino-thiazol-4-yl, 2-methylthiazol-4-yl, 6-chloro-pyridin-2-yl, 6-amino-pyridin-2-yl, etc..

The presence of at least one asymmetric carbon atom in the compounds of formula I means that the compounds may occur in optically isomeric and enantiomeric forms. As a result of the presence of a possible aliphatic C=C double bond, geometric isomerism may also occur. Formula I is intended to include all those possible isomeric forms and mixtures thereof.

Certain compounds of formula I are capable of forming acid addition salts, for example with inorganic acids, such as hydrochloric acid, sulfuric acid or a phosphoric acid, or with suitable organic carboxylic or sulfonic acids, for example aliphatic mono- or dicarboxylic acids, such as trifluoroacetic acid, acetic acid, propionic acid, glycolic acid, succinic acid, maleic acid, fumaric acid, hydroxymaleic acid, maleic acid, tartaric acid, citric acid, oxalic acid or amino acids, such as argentine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxy-ethane-sulfonic acid, or aromatic sulfonic acids, for example benzene-, p-toluene- or naphthalene-2-sulfonic acid.

Formula I according to the invention shall include all the possible tautomers, isomeric forms, as well as mixtures, e.g. racemic mixtures, and any mixtures of rotamers.

In view of the close relationship between the compounds of formula I in free form and in the form of their salts, including also salts that can be used as intermediates, for example in the purification of the compounds of formula I or in order to identify those compounds, herein-before and hereinafter any reference to the (free) compounds is to be understood as including also the corresponding salts, where appropriate and expedient.

Among the compounds of formula I according to the present invention the following groups of compounds are preferred. These groups are those wherein
R₁ is hydrogen, COCH₃, COCH₂CH₃, SO₂N(CH₃)₂, COOCH₃, COOCH₂CH₃, CON(CH₃)₂, SO₂CH₃, CH₂C≡CH, methyl, ethyl, n-propyl, n-butyl; or
R₁ is hydrogen, COCH₃, COCH₂CH₃, SO₂N(CH₃)₂, COOCH₃ or COOCH₂CH₃; or R₁ is COCH₃, COCH₂CH₃, SO₂N(CH₃)₂, COOCH₃ or COOCH₂CH₃; or
R₁ is hydrogen, or
R₂ is hydrogen or NH₂; or
R₂ is hydrogen; or
R₃ is phenyl optionally substituted by one, two or three substituents R₁₂ wherein each of the R₁₂ groups may be the same or different; or
R₃ is phenyl optionally substituted by one substituent R₁₂ ; or
R₃ is phenyl optionally para-substituted by one R₁₂ group; or
R₃ is phenyl optionally para-substituted by fluoro, chloro, bromo, methyl, ethyl, methoxy or ethoxy, or
R₄ is hydrogen, hydroxy, cyano, fluorine, chlorine, bromine, 2-hydroximino-ethyl, 2-methoximino-ethyl,C₁-C₄alkyl, C₁-C₄alkoxy, C₁-C₄haloalkoxy, C₁-C₄haloalkyl, C₁-C₄cyanoalkyl, C₁-C₄alkanoyloxy, C₁-C₄hydroxyalkyl, C₁-C₄haloalkanoyloxy, C₁-C₄alkanoyl-C₁-C₆aminoalkyl, C₁-C₄alkanoyloxy-C₁-C₄alkyl, C₁-C₄alkanoyl, C₁-C₄alkylthio, C₁-C₄alkoxycarbonyl, heteroaryl or heterocyclyl, C₁-C₄alkanoyloxy, C₁-C₄alkyloxycarbonyloxy, C₁₋C₄alkanoyloxy, di(C₁-C₄alkyl)aminoC₁-C₄alkanoyloxy or di(C₁-C₄alkyl)aminosulfonyloxy; or R₄ is hydrogen, hydroxy, cyano, fluorine, chlorine, bromine, methyl, tert. butyl, methylthio, trifluoromethyl, cyanomethyl, 2-cyanoethyl, hydroxymethyl, 1-hydroxyethyl, 2-hydroxyisopropyl, acetyl, 2-hydroximino-ethyl, 2-methoximino-ethyl, acetoxymethyl, methoxycarbonyl, methoxy, ethoxy or trifluoromethoxy oxadiazolyl (optionally substituted by methyl or trifluoromethyl), piperidinyl, morpholinyl, piperazinyl, pyridyloxy, pyrimidmyloxy, thiazolyloxy, pyridazinyloxy, C₁-C₄alkanoyloxy, C₁-C₄alkyloxycarbonyloxy, C₁₋C₄alkanoyloxy, di(C₁-C₄alkyl)aminoC₁-C₄alkanoyloxy or di(C₁-C₄alkyl)aminosulfonyloxy, or R₅ is hydrogen, methyl, methoxy or halogen; or
R₅ is hydrogen; or
R₆ is hydrogen, methyl, methoxy or halogen; or
R₆ is hydrogen; or
R₇ is hydrogen, methyl, methoxy or halogen; or
R₇ is hydrogen.

Further preferred subgroups of formula I are those compounds wherein:
a) R₂, R₅, R₆ and R₇ are all hydrogen, or
b) R₁ is hydrogen or -CO-lower alkyl, -COO-lower alkyl or SO₂N(lower alkyl)_{2;} R₃ is phenyl optionally substituted by one substituent R₁₂;and R₄ is hydrogen, hydroxy, cyano, C₁₋C₄alkyl, C₁-C₄alkoxy, C₁-C₄haloalkoxy, C₁-C₄haloalkyl, C₁-C₄cyanoalkyl, C₁-C₄alkanoyloxy, C₁-C₄hydroxyalkyl, C₁-C₄haloalkanoyloxy, C₁-C₄alkanoyl-C₁-C₆aminoalkyl, C₁₋C₄alkanoyloxy-C₁-C₄alkyl, C₁-C₄alkanoyl, C₁-C₄alkylthio, C₁-C₄alkoxycarbonyl, heteroaryl or heterocyclyl, C₁-C₄alkanoyloxy, C₁-C₄alkyloxycarbonyloxy, C₁-C₄alkanoyloxy, di(C₁₋C₄allcyl)aminoC₁-C₄alkanoyloxy or di(C₁-C₄alkyl)aminosulfonyloxy; or
c) R₁ is COCH₃, COCH₂CH₃, SO₂N(CH₃)₂, COOCH₃ or COOCH₂CH₃; R₃ is phenyl optionally para-substituted by one R₁₂ group and R₄ is hydrogen, hydroxy, cyano, fluorine, chlorine, bromine, methyl, tert. butyl, methylthio, trifluoromethyl, cyanomethyl, 2-cyanoethyl, hydroxymethyl, 1-hydroxyethyl, 2-hydroxyisopropyl, acetyl, 2-hydroximino-ethyl, 2-methoximino-ethyl, acetoxymethyl, methoxycarbonyl, methoxy, ethoxy or trifluoromethoxy oxadiazolyl (optionally substituted by methyl or trifluoromethyl), piperidinyl, morpholinyl, piperazinyl, pyridyloxy, pyrimidinyloxy, thiazolyloxy, pyridazinyloxy, C₁-C₄alkanoyloxy, C₁-C₄alkyloxycarbonyloxy, C₁-C₄alkanoyloxy, di(C₁₋C₄alkyl)aminoC₁-C₄alkanoyloxy or di(C₁-C₄alkyl)aminosulfonyloxy.

Other preferred subgroups are characterized by subformula IA 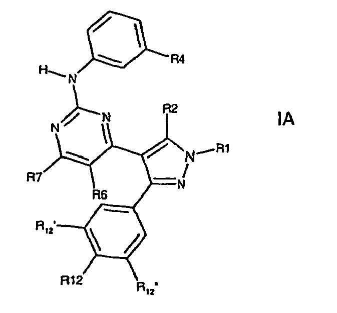 wherein R₁, R₂, R₄, R₆, R₇ are as defined in relation to Formula I and R₁₂, R_{12'} and R_{12''} are independently as defined for R₁₂ in formula I; or
by subformula IB 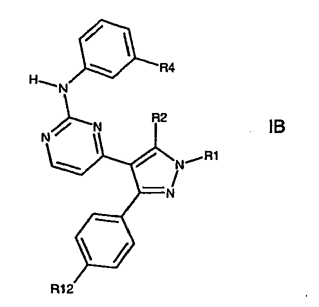 wherein R₁, R₂, R₄ and R₁₂ are as defined in relation to Formula I.

The compounds according to the invention may be prepared according to methods *per se* known in the art (this means that in spite of employing generally known types of organic reaction steps, that where novel compounds are produced, the respective process of manufacture and the sequence of steps is novel because it has not been known for be employable for the novel compound). The procedures for the preparation of compounds of formula I may be outlined as follows:

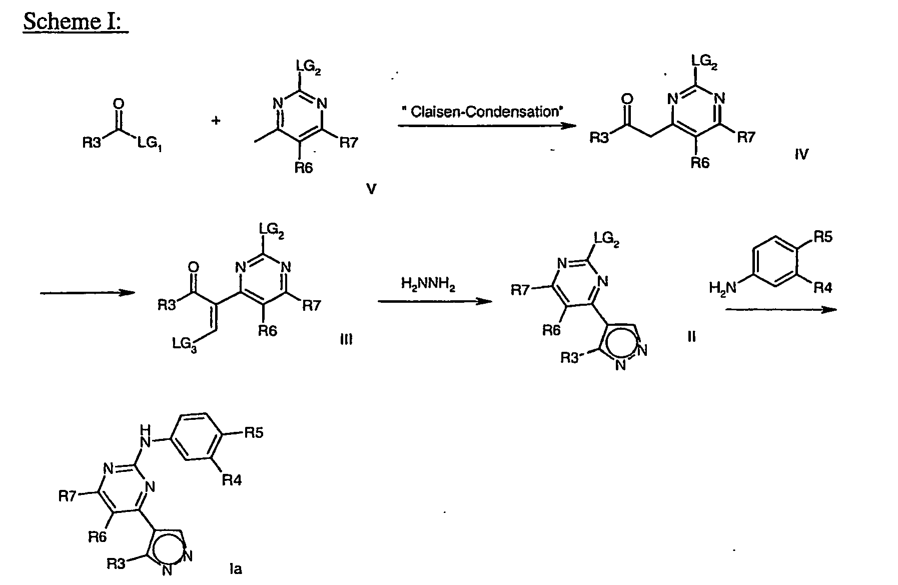

Compounds of the general formula Ia may be obtained by reacting a compound of the general formula II with an aniline. The nucleophilic displacement of the group LG₂ is typically conducted in the presence of a Lewis acid using an excess of aniline. LG₂ stands for a leaving group, such as halogen, alkylsulfide, alkylsulfoxide or alkylsulfone. The latter two leaving groups are conveniently obtained by oxidation of the corresponding alkylsulfide using standard reaction conditions. Typical Lewis acids include boron trifluoride, zinc halogenides and sulfonic acids.

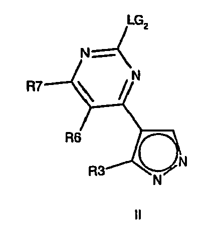

The reaction may be carried out in an inert solvent, such as an ether e.g. tetrahydrofuran, dioxane, diglyme and the like. The reaction temperature may vary within ranges, e.g. from 20°C to 220°C.

The anilines are either commercially available or can be synthesized using standard procedures.

Compounds of the general formula II may be obtained by reacting a compound of formula III with hydrazine or a hydrated form thereof. Inert solvents such as ethers, alcohols and the like may be used. LG₃ stands for a leaving group such as hydroxy, alkoxy, dialkylamino and the like. The reaction temperature may be varied but typically room temperature is sufficient.

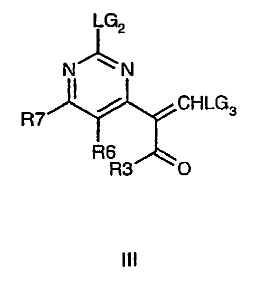

Compounds of the general formula III are obtained by reacting a compound of the general formula IV with a formylating agent such as an alkyl formiate or an acetal of N,N-dialkylformamide. The latter method is the preferred one. A solvent may be used if desired. Typical solvents include alcohols such as ethanol, esters such as ethyl acetate and also more polar solvents such as DMF, NMP and the like. The reaction temperature lies in the range of room temperature to the boiling point of the reaction mixture.

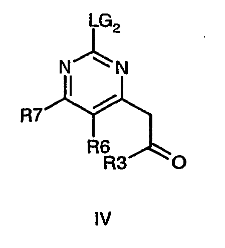

Compounds of the general formula IV may be prepared by reacting a corresponding methylpyrimidine with an ester, dialkylamide or N,O-dialkylamide of R₃COOH under the influence of a strong base. Typical bases used in this reaction are alkoxides such as potassium t-butoxide or sodium t-butoxide, lithiumamides, such as LDA or LiHMDS and metal hydrides, such as potassium or sodium hydride. Typical solvents are ethers, such as diethylether, glyme and THF. In certain cases the use of more polar solvents, such as DMF, DMSO and HMPT is preferred. The reaction is typically performed at -78°C to the boiling point of the solvent.

Aminopyrazoles of the general formula Ib may be prepared according to the *scheme 2*. The oxidation/displacement step of the leaving group in the 2-position of the pyrimidine is already described above (cf. *scheme 1*).

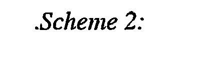

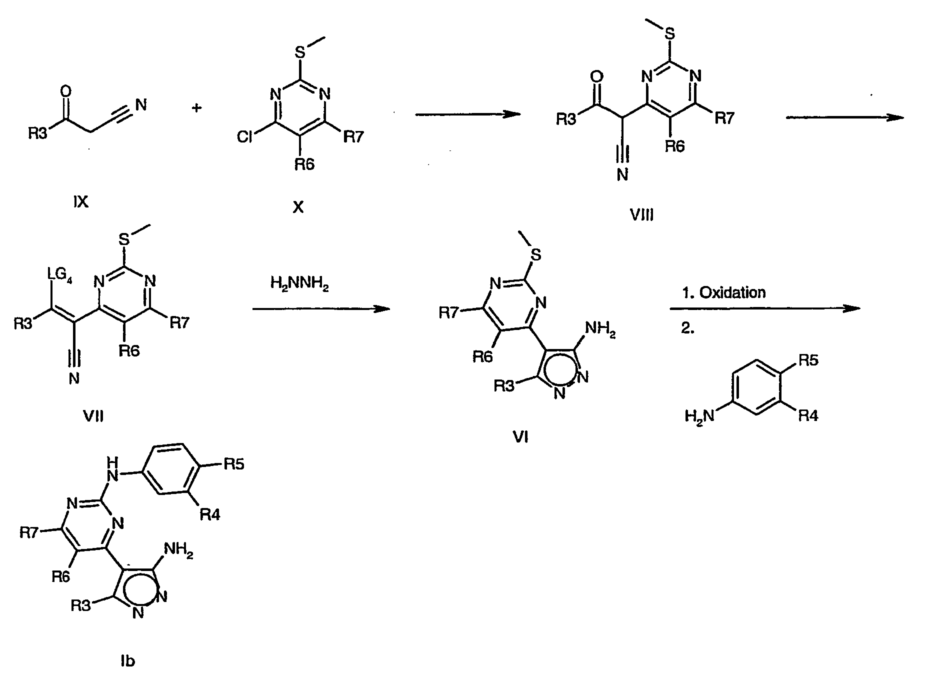

Compounds of the general formula VI can be prepared by cyclization of a compound of the general formula VII with hydrazine.

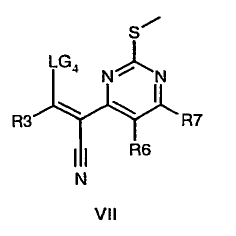

LG₄ represents halogen such as chlorine or bromine, O-alkyl- or arylsulfonyl such as methylsulfonyl or p-toluolylsulfonyl. Hydrazine may be used as solvent. Other solvents such as alcohols like ethanol or isopropanol may be used if desired. The reaction temperature lies in the range of room temperature to the boiling point of the solvent.

Compounds of the general formula VII may be prepared by reacting a cyanoketone VDI with a halogenating agent such as POCl₃, PCl₃, SOCl₂, oxalyl chloride and the like or by a sulfonylating agent such as methylsulfonylchloride or p-toluolylsulfonylchloride. Typical solvents for this reaction embrace halogenated hydrocarbons such as methylene chloride, aromatics such as toluene and others. The reaction is catalyzed by dialkylformamides, such as N,N-dimethylformamide. The reaction temperature lies close to room temperature but may be as high as the boiling point of the reaction mixture.

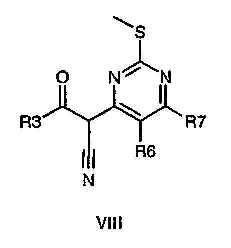

Cyano ketones of the general formula VIII may be conveniently prepared by the reaction of a cyanoketone IX with a compound of the general formula X. At least two equivalents of a mild base are required to drive the reaction to completion. Typical bases include alkaline metal carbonates and/or bicarbonates such as sodium carbonate, potassium carbonate, cesium carbonate and potassium bicarbonate. The reaction may be performed in the presence of a diluting agent. Typical diluting agents comprise alcohols such as methanol, ethanol and higher boiling alcohols, ketones such as acetone and other polar solvents such as N,N-dimethylformamide, dimethylsulfoxide, NMP and others. Alternatively the cyano ketones may be prepared as outlined as follows:

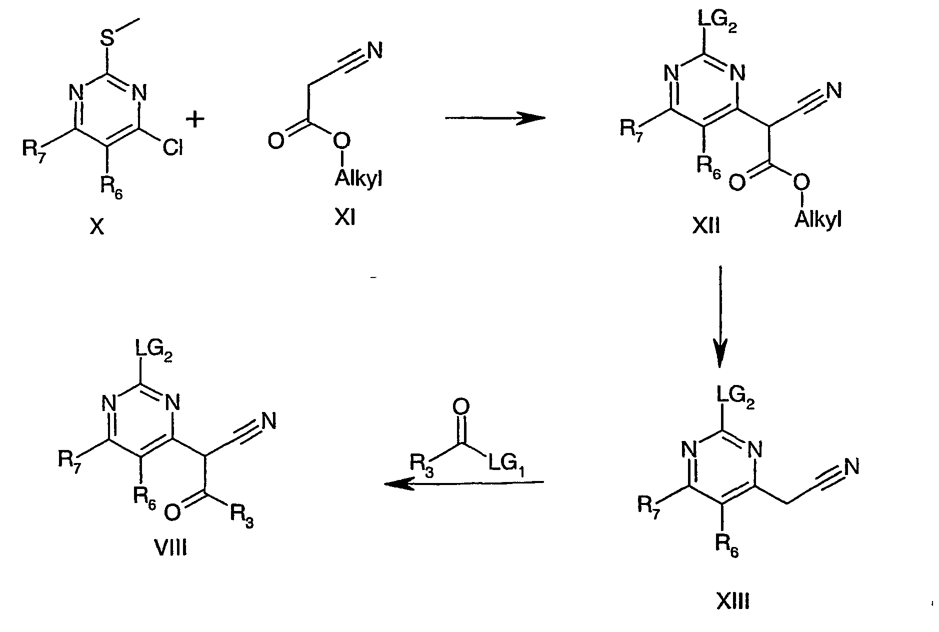

Compounds of formula X are reacted with compounds of formula XI where alkyl stands for e.g. methyl, ethyl, t-butyl, preferably t-butyl, in presence of a base and a suitable solvent to yield compounds of formula XII. Typical bases include alkaline metal carbonates and/or bicarbonates such as sodium carbonate, potassium carbonate, cesium carbonate and potassium bicarbonate. The reaction may be performed in the presence of a diluting agent and a temperature range between room temperature and the boiling point of the solvent, preferably between 90 -100 °C. Typical diluting agents comprise ketones such as acetone and other polar solvents such as N,N-dimethylformamide, dimethylsulfoxide, NMP and others. The reaction may be catalysed by addition of alkaline metal iodides such as potassium iodide or 4-dialkylaminopyridines such as 4-dimethylamino-pyridine. Compounds of formula XII are subjected to hydrolysis using strong acids in a solvent to yield compounds of formula XIII. Typical strong acids are hydrogen halides , such hydrogen chloride, hydrogen bromide, hydrogen iodide. Typical solvents are alcohols such as ethanol, methanol, iso-propanol or organic acids such as acetic acid. The reaction temperature may be varied between room temperature and 100 °C and is typically around 50 °C. Compounds of formula VIII are obtained by benzoylating compounds of formula XIII with R₃CO-LG₁ in the presence of a base in a suitable solvent. Typical bases are alkaline alkoholates such as sodium or potassium methoxide, sodium or potassium ethoxide or magnesium methoxide or magnesium ethoxide, preferably the latter. Suitable solvents are lower alcohols and ethers such as tetrahydrofuran, dioxane and the like. The reaction may be performed at a temperature of 50-100°C.

Compounds of the general formula IX and X are either commercially available or can be prepared according to known methods.

In the case of X=alkylthio the reactivity may be enhanced by oxidizing the leaving group to the corresponding sulfoxide or sulfone using standard conditions for such transformations.

For various reasons it may also be desirable to transform one derivative of formula I into another one which is also within the general definition of formula I. Thus, for example it is possible to acylate compounds of the formula I using acylating agents to prepare compounds of the general formula Ic.:

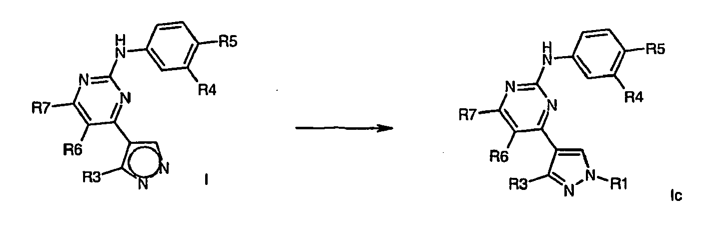

Typical acylating agents for this purpose include carboxylic acid chlorides, carboxylic acid anhydrides, chloroalkyl formiates, carbamoylchlorides, sulfamoylchlorides, sulfonylchlorides and the like. The acylation may be carried out in the presence or absence of a base. The reaction temperature will suitably lie in the range from 0°C to the boiling point of the reaction mixture. Inert solvents may be used where desired or indicated by the nature of the reagents. Examples of suitable bases include alkaline metal carbonates or bicarbonates, such as potassium carbonate or sodium hydrogen carbonate (referred to as *Schotten-Baumann conditions)* or tertiary amines, such as triethylamine, DABCO or diisopropylethylamine. Examples of suitable solvents include aromatic hydrocarbons such as toluene; ethers such as diethyl ether, tetrahydrofuran and glyme or mixtures with water. Regioisomers may result. These can be separated using known methods.

Reactive groups R₄ and/or R₅ in the starting materials I, such as hydroxy, amino, hydroxyalkyl, aminoalkyl and the like may tend to react preferably to give acylated products. Such intermediates may be isolated and are of interest as such or they may be further reacted with a second equivalent of acylating agent.

Derivatives of the subformula Id 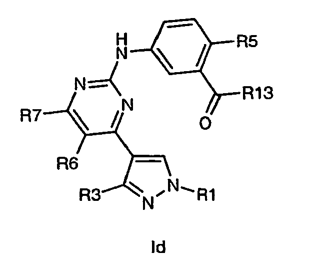 may be modified if desired at the functional carbonylic group, e.g. with Grignard-reagents and other metallated radicals to yield the corresponding tertiary alcohols. The reaction of this type is routinely performed in inert solvents and with using an excess of metallated agent. Typical solvents are ethers such as diethyl ether, tetrahydrofuran, glyme and the like. The reaction temperature lies in the range of 0°C to the boiling point of the solvent, but is normally kept under strict control by cooling and slow addition of the reactants. The standard work-up processes allow isolation of the compounds of formula I, wherein R₄ is hydroxyalkyl in high yields and as pure compounds.

When employing the functional intermediate of formula If 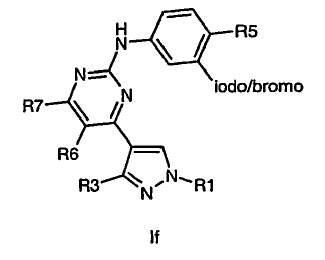 additional transition metal catalyzed reactions, such as *Sonogashira, Heck, Stille* and *Suzuki* couplings, as well as *Hartwig-Buchwald* aminations offer further opportunities for derivatization of already prepared compounds of formula I. The indicated methods are known in the art and may be suitably adapted to the requirements of the envisaged derivatization. Furthermore intermediates of the general formula Ig wherein L stands for a direct bond or a spacing group 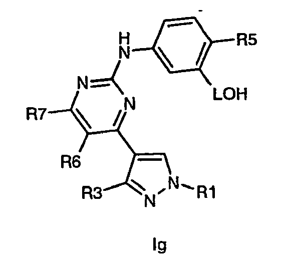 may be selectively alkylated/acylated at the phenolic hydroxy group. This derivatization is of special importance as several targeted compounds can not be prepared by the general route outlined in *scheme 1.*

In addition to the novel active fungicidal compounds the present invention also relates to novel starting materials and/or intermediates and to processes for the preparation thereof. The starting materials used and the reaction conditions chosen are preferably such that the compounds shown in this disclosure as being especially preferred or to be used preferably are obtained. Especially preferred among the process conditions are those described in the examples below, or analogous procedures.

The invention also relates to compositions which comprise the compounds of the formula I, or a salt thereof, as an active component, in particular plant-protecting compositions, and also to their use in the agricultural sector or related areas.

Active compounds of the formula I are customarily used in the form of compositions and may be added, simultaneously or successively, to the surface or plant to be treated together with additional active compounds. These additional active compounds may be either fertilizers, trace element-supplying agents or other preparations which influence plant growth. It is also possible, in this context, to use selective herbicides, such as insecticides, fungicides, bactericides, nematicides or molluscicides, or mixtures of several of these preparations, additionally, where appropriate, together with excipients, surfactants or other administration-promoting additives which are customary in formulation technology (designated collectively as carrier materials herein).

Suitable excipients and additives may be solid or liquid and are those substances which are appropriate in formulation technology, for example natural or regenerated minerals, solvents, dispersants, wetting agents, adhesives, thickening agents, binding agents or fertilizers.

A preferred method for applying a compound of formula I, or an agrochemical composition which comprises at least one of these compounds, is administration to the leaves (foliar application). The frequency and rate of administration depend upon the risk of infestation by the corresponding pathogen. The compounds of formula I can, however, also penetrate the plant through the roots via the soil (systemic action). If the locus of the plant is impregnated with a liquid formulation or if the substances are introduced in solid form into the soil, e.g. in the form of granules (soil application). In paddy rice crops, such granules can be applied in metered amounts to the flooded rice fields. In order to treat seeds , the compounds of formula I can, however, also be applied to the seeds (coating), either by impregnating the grains or tubers with a liquid formulation of the active ingredient, or by coating them with a solid formulation.

Advantageous rates of application are in normally from 5 g to 2 kg of active ingredient (a.i.) per hectare (ha), preferably from 10 g to 1 kg of a.i./ha, especially from 20 g to 600 g a.i./ha. When the compound are used as seed dressings, dosages of from 10 mg to 1 g of active ingredient per kg seed are advantageous employed. The agrochemical compositions generally comprise 0.1 to 99% by weight, preferably 0.1 to 95% by weight, of a compound of formula I, 99.9 to 1% by weight, preferably 99.8 to 5% by weight, of a solid or liquid adjuvant and 0 to 25% by weight, preferably 0.1 to 25 % by weight, of a surfactant. Whereas commercial products will preferably be formulated as concentrates, the end user will normally employ dilute formulations.

The compositions may also comprise further auxiliaries, such as fertilizers and other active ingredients for obtaining special desirable biological effects.

The compounds of formula I may be used preventatively and/or curatively in the sector of agronomics and related technical areas as active ingredients for controlling plant pests. The active ingredients of formula I according to the invention are notable for their good activity even at low concentrations, for their good plant tolerance and for their environmentally friendly nature. They have very advantageous, especially systemic, properties and may be used to protect a plurality of cultivated plants. Using the active ingredients of formula I on plants or plant parts (fruit, flowers, leaves, stems, tubers, roots) of various crops, the pests appearing can be controlled or destroyed, whereby the parts of plants which grow later also remain protected, e.g. from phytopathogenic microorganisms.

The compounds I may additionally be used as a dressing to treat seeds (fruits, tubers, corms) and plant cuttings to protect against fungal infections and against phytopathogenic fungi occurring in the soil.

The compounds I are effective for example against the following classes of related phytopathogenic fungi: *Fungi imperfecti* (e.g*. Botrytis, Pyricularia, Helminthosporium, Fusarium, Septoria, Cercospora* and *Altenzaria); Basidiomycetes* (e.g. *Rhizoctonia, Hemileia, Puccinia); Ascomycetes* (e.g. *Venturia* and *Erysiphe, Podosphaera, Monilinia, Uncinula)* and *Oomycetes* (e.g. *Phytophthora, Pythium, Plasmopara).*

Target crops for the plant-protecting usage in terms of the invention are for example the following plant cultivars: cereals (wheat, barley, rye, oats, rice, maize, sorghum and related species); beet (sugar beet and fodder beet); pome, stone and berry fruit (apples, pears, plums, peaches, almonds, cherries, strawberries, raspberries and blackberries); legumes (beans, lentils, peas, soya); oil crops (rape, mustard, poppy, olives, sunflowers, coconut, castor oil, cocoa, peanut); cucumber plants (squashes, cucumber, melons); citrus fruits (oranges, lemons, grapefruits, mandarines); vegetables (spinach, lettuce, asparagus, cabbage varieties, carrots, onions, tomatoes, potatoes, paprika); laurels (avocado, cinnamonium, camphor) and plants such as tobacco, nuts, coffee, aubergines, sugar cane, tea, pepper, vines, hops, bananas and natural rubber plants, as well as ornamental plants.

In particular the compounds of formula I have good activity against the oomycetes in grapes, potatoes and vegetables.

Further areas of application for the active ingredients according to the invention are the protection of stores and material, where the storage matter is protected against putrescence and mould.

The compounds I are used in unchanged form or preferably together with customary excipients in formulation techniques. To this end, they are conveniently processed in known manner e.g. into emulsion concentrates, coatable pastes, directly sprayable or diluable solutions, diluted emulsions, wettable powders, soluble powders, dusts or granules, e.g. by encapsulation into for example polymeric materials. As with the type of medium, the application processes, such as spraying, atomizing, dusting, scattering, coating or pouring are similarly chosen according to the desired aims and the prevailing conditions.

Suitable substrates and additives may be solid or liquid and are useful substances in formulation techniques, e.g. natural or regenerated mineral substances, dissolving aids, dispersants, wetting agents, tackifiers, thickeners or binding agents.

The compounds of formula I may be mixed with further active ingredients, e.g. fertilizers, ingredients providing trace elements or other active ingredients used in the plant protection science, especially further fungicides. In doing so, in some cases synergistic enhancement of the biological effects may occur.

Preferred active ingredients advantageous as additives to the compositions comprising the active ingredient of formula I are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, pyrifenox, prochloraz, propiconazole, simeconazole, tebuconazole, tetraconazole, triadimefon, triadimenol, triflumizole, triticonazole; pyrimidinyl carbinoles, such as ancymidol, fenarimol, nuarimol; 2-amino-pyrimidines, such as bupirimate, dimethirimol, ethirimol; morpholines, such as dodemorph, fenpropidine, fenpropimorph, spiroxamine, tridemorph; anilinopyrimidines, such as cyprodinil, mepanipyrim, pyrimethanil; pyrroles, such as fenpiclonil, fludioxonil; phenylamides, such as benalaxyl, furalaxyl, metalaxyl, R-metalaxyl, ofurace, oxadixyl; benzimidazoles, such as benomyl, carbendazim, debacarb, fuberidazole, thiabendazole; dicarboximides, such as chlozolinate, dichlozoline, iprodione, myclozoline, procymidone, vinclozoline; carboxamides, such as carboxin, fenfuram, flutolanil, mepronil, oxycarboxin, thifluzamide; guanidines, such as guazatine, dodine, iminoctadine; strobilurines, such as azoxystrobin, kresoxim-methyl, metominostrobin, SSF-129, trifloxystrobin, picoxystrobin, BAS 500F (proposed name pyraclostrobin), BAS 520; dithiocarbamates, such as ferbam, mancozeb, maneb, metiram, propineb, thiram, zineb, ziram; N-halomethylthiotetrahydrophthalimides, such as captafol, captan, dichlofluanid, fluoromides, folpet, tolyfluanid; Cu-compounds, such as Bordeaux mixture, copper hydroxide, copper oxychloride, copper sulfate, cuprous oxide, mancopper, oxine-copper; nitrophenol-derivatives, such as dinocap, nitrothal-isopropyl; organo-p-derivatives, such as edifenphos, iprobenphos, isoprothiolane, phosdiphen, pyrazophos, tolclofos-methyl; various others, such as acibenzolar-S-methyl, anilazine, benthiavalicarb, blasticidin-S, chinomethionate, chloroneb, chlorothalonil, cyflufenamid, cymoxanil, dichlone, diclomezine, dicloran, diethofencarb, dimethomorph, SYP-LI90 (proposed name: flumorph), dithianon, ethaboxam, etridiazole, famoxadone, fenamidone, fenoxanil, fentin, ferimzone, fluazinam, flusulfamide, fenhexamid, fosetyl-aluminium, hymexazol, iprovalicarb, IKF-916 (cyazofamid), kasugamycin, methasulfocarb, metrafenone, nicobifen, pencycuron, phthalide, polyoxins, probenazole, propamocarb, pyroquilon, quinoxyfen, quintozene, sulfur, triazoxide, tricyclazole, triforine, validamycin, zoxamide (RH7281).

One preferred method of application of an active ingredient of formula I or of an agrochemical composition containing at least one of these active ingredients is foliar application. The frequency and amount of application depend on the severity of the attack by the pathogen in question. However, the active ingredients I may also reach the plants through the root system via the soil (systemic action) by drenching the locus of the plant with a liquid preparation or by incorporating the substances into the soil in solid form, e.g. in the form of granules (soil application). In rice cultivations, these granules may be dispensed over the flooded paddy field. The compounds I may however also be applied to seed grain to treat seed material (coating), whereby the grains or tubers are either drenched in a liquid preparation of the active ingredient or coated with a solid preparation.

The compositions are produced in known manner, e.g. by intimately mixing and/or grinding the active ingredient with extenders such as solvents, solid carriers and optionally surfactants.

Favorable application rates are in general 1 g to 2 kg of active substance (AS) per hectare (ha), preferably 10 g to 1 kg AS/ha, especially 20 g to 600 g AS/ha. For usage as a seed dressing, it is advantageous to use dosages of 10 mg to 1 g active substance per kg of seed grain.

While concentrated compositions are preferred for commercial usage, the end user normally uses diluted compositions.

Formulations may be prepared analogously to those described for example in WO 97/33890.

### Examples:

The subsequent examples are intended to illustrate the invention, without however limiting the scope thereof.

### Example 1:

### [4-(3-Amino-5-phenyl-1H-pyrazol-4-yl)-pyrimidin-2-yl]-phenyl-amine

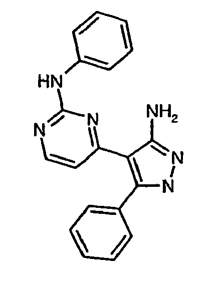

A mixture of 4-(2-methanesulfinyl-pyrimidin-4-yl)-5-phenyl-1H-pyrazol-3-ylamine (0.61g, 2.0mmol) and aniline (1.9g, 20mmol) was heated at 100°C. After the addition of boron trifluoride diethyl etherate (3 drops) the solution was heated at 100°C for an hour. The reaction mixture was poured while still hot onto crushed iced. The product was extracted repeatedly with mixtures of ethyl acetate and tetrahydrofuran. The pooled organic solutions were dried and the solvents removed on a rotary evaporator. After chromatography (eluent: ethyl acetate / hexane) the title compound was obtained as colorless solid (m.p. 265-266°C).

### 4-(2-Methanesulfinyl-pyrimidin-4-yl)-5-phenyl-1H-pyrazol-3-ylamine

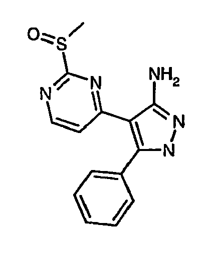

A suspension of 4-(2methanesulfanyl-pyrimidin-4-yl)-5-phenyl-1H-pyrazol-3-ylamine (1.1g, 3.9mmol) in methylene chloride (30ml) was cooled to 0°C. After the addition of m-chloro perbenzoic acid (1.05g, content of 70% peracid, 4.2mmol) the resulting solution was stirred for an additional hour. The reaction mixture was neutralized by addition of a saturated aqueous solution of sodium bicarbonate. The reaction mixture was diluted with enough ethyl acetate to give two phases. The organic phase was separated, dried and evaporated under reduced pressure to give the title compound in form of slightly colored crystals.

### 4-(2-Methanesulfanyl-pvrimidin-4-yl)-5-phenyl-1H-pyrazol-3-ylamine

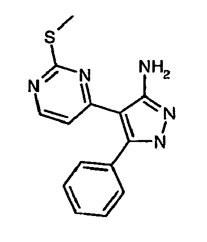

A suspension of 2-(2-methylsulfanyl-pyrimidin-4-yl)-3-oxo-3-phenyl-propionitrile (10.2g, 38mmol) methylene chloride (150ml) was treated with a catalytic amount of dimethylformamide followed by oxalyl chloride (3.75ml, 38mmol). After 1hour at room temperature the solvent was evaporated under reduced pressure to give the intermediate (3-chloro-2-(2-methylsulfanyl-pyrimidin-4-yl)-3-phenyl-acrylonitrile as an oil. The product was dissolved in ethanol (20ml) and added dropwise over a period of 20 minutes to a heated solution of hydrazine hydrate (15ml,) in ethanol (100ml) at 50°C. The mixture was refluxed for 3 hours followed by evaporation of the solvent under reduced pressure. The residue was purified on silicagel using mixtures of ethyl acetate and hexanes (1:1) to give the title compound in form of a colorless oil. ¹H-NMR: 12.30 (s, br, 1H); 8.34 (d, 1H); 7.73-7.52 (m, 5H); 6.70 (s, br, 2H); 6.62 (d, 1H); 3.50 (s, 3H).

### 2-(2-Methylsulfanyl-pyrimidin-4-yl)-3-oxo-3-phenyl-propionitrile

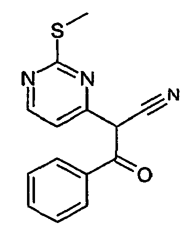

A suspension of benzoylacetonitrile (11.0g, 0.076mol) and potassium carbonate (20.7g, 0.15mol) in N-methylpyrrolidon (50ml) was heated at 85°C. A solution of 2-methylmercapto-4-chloro-pyrimidine (14.4g, 0.09mol) in N-methylpyrrolidon (10ml) was added dropwise within 30 minutes. The reaction mixture was heated for another 15 hours at this temperature.
The mixture was cooled to room temperature and poured carefully onto a mixture of crushed ice and concentrated hydrochloric acid. The resulting precipitate is filtered with suction and dried under vacuum at 100°C to give the title compound as colorless crystals showing a m.p. of 163-164°C.

### Example 2:

### [4-(5-phenyl-1H-pyrazol-4-yl)-pirimidin-2-yl]-N-[(3-cyanomethyl)-phenyl]-amine

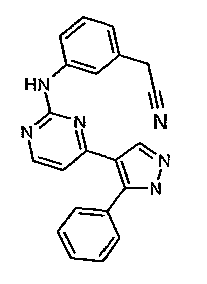

A mixture of 4-(2-methanesulfinyl-pyrimidin-4-yl)-5-phenyl-1H-pyrazol (0.6g, 1.76mmol) and 3-cyanomethylaniline (2.0g, 15.2mmol) was heated at 100°C. After the addition of boron trifluoride diethyl etherate (3 drops) heating at the same temperature was continued for half an hour. The starting material started dissolving yielding a clear solution, followed by the precipitation of the title compound as a crystalline mass. The reaction mixture was diluted with diethyl ether. The crystals are filtered and washed thoroughly with diethyl ether to give the product in form of colorless crystals, having a m.p. of 230-231 °C.

### 2-Methanesulfinyl-4-(5-phenyl-1H-pyrazol-4-yl)-pyrimidine

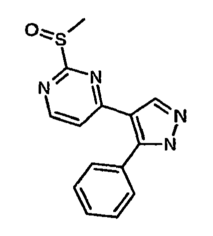

A suspension of 4-(2-methanesulfanyl-pyrimidin-4-yl)-5-phenyl-1H-pyrazol-3-ylamine (3.0g, 11.8mmol) in methylene chloride (100ml) was cooled to 0°C. After the addition of m-chloro perbenzoic acid (3.2g, content of 70% peracid, 13.0mmol) the resulting solution was stirred for an additional hour. The reaction mixture was neutralized by addition of a saturated aqueous solution of sodium bicarbonate. The reaction mixture was diluted with enough ethyl acetate to give two phases. The organic phase is separated, dried and evaporated under reduced pressure to give the title compound in form of slightly colored crystals.

### 4-(2-Methanesulfanyl-pyrimidin-4-yl)-5-phenyl-1H-pyrazol-3-ylamine

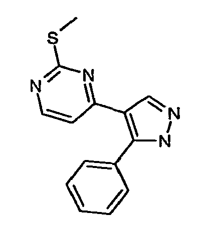

A mixture of 4-methyl-2-methylsulfanyl-pyrimidine (30g, 0.21mol) and methyl benzoate (30g, 0.21mol) was added to a solution of potassium t-butoxide (54g, 0.48mol) in tetrahydrofurane (350ml) with cooling in such a way that the reaction temperature did not exceed 20°C. After stirring the mixture for additional 20 minutes the solution was poured onto crushed ice. The resulting solution was acidified with concentrated hydrochloric acid and extracted with ethyl acetate. Drying of the organic phase, filtering and evaporation of the solvent under reduced pressure gave an oil, that was sufficiently pure for the next step. The intermediate prepared in the first step was heated at reflux with N-N-dimethylformamide diethylacetal (56ml, 0.32mol) for 2 hours. The excess reagent was allowed to distil off and the residual oil was dissolved in ethanol (80ml). To this solution was added hydrazine hydrate (20ml) with cooling. After stirring for 2 hours at room temperature water was added with cooling until all product had separated in crystalline form. Filtration of the product with suction, washing with diethylether and drying at 100°C under high vacuum gave the title compound as an almost colorless solid, m.p. of 165-166°C.

### Example 3:

### [4-(5-p-Fluoro-phenyl-1H-pyrazol-4-yl)-pyrimidin-2-yl]-N-[3-(2-hydroxy-2-methyl-ethyl)-phenyl]-amine

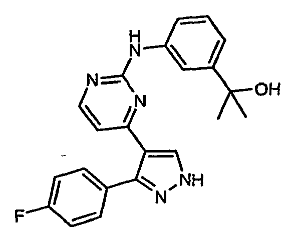

a)[4-(5-p-Fluoro-phenyl-1H-pyrgol-4-yl)-pyrimidin-2-yl]-N-(3-methoxycarbonyl-phenyl)-amine 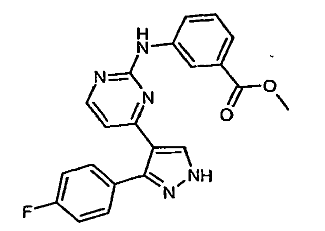
   A mixture of 4-(2-methanesulfinyl-pyrimidin-4-yl)-5-p-fluorphenyl-1H-pyrazol (3.0g, 9.9mmol) and m-amino benzoic acid methyl ester (15.0g, 99mmol) was heated at +100°C. After the addition of boron trifluoride diethyl etherate (3 drops) the mixture was heated at 150°C for half an hour. The reaction mixture was poured while still hot onto crushed iced. Diethylether was added to the resulting semi crystalline residue and the mixture stirred vigourously before filtering. The product was obtained as colorless solid, showing a m.p. of > 265°C after being dried at high vacuum.
b) To a suspension of [4-(5-phenyl-1H-pyrazol-4-yl)-pyrimidin-2-yl]-N-(3-methoxycarbonyl-phenyl)-amine (1.0g, 2.6mmol) and a catalytic amount of CeCl₃ in tetrahydrofuran (50ml) was added a solution of methyl magnesium chloride (6ml of a 20% THF solution) controlling the temperature so as not to exceed 15°C. After stirring the mixture for an additional hour the solution was poured onto crushed ice and ammonium chloride. The resulting suspension was extracted repeatedly with ethyl acetate. The organic phases were pooled, dried and concentrated under reduced pressure. Chromatography of the resulting solid gives the title compound as a colorless solid, m.p. 263-264°C.

### Example 4:

### [4-(5-Phenyl-1-acetyl-pyrazol-4-yl)-pyrinfldin-2-yl]-N-(3-acetoxymethyl-phenyl)-amine

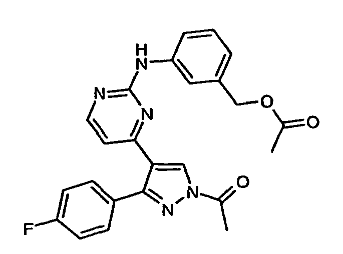

To a suspension of [4-(5-p-fluoro-phenyl-1H-pyrazol-4-yl)-pyrimidin-2-yl]-N-(3-hydroxymethyl-phenyl)-amine (1.0g, 2.8mmol) in THF (20ml) was added sequentially N,N-dimethylaminopyridine (1g,) and acetic acid anhydride (1ml). The resulting solution was stirred for 16 hours at room temperature. The mixture was diluted with ethyl acetate and washed with brine. Drying, evaporation of the solvents and purification on silicagel gives the title compound as a colorless solid, m.p. 133-134°C.

### [4-(5-p-Fluor-phenyl-1H-pyrazol-4-yl)-pyrimidin-2-yl]-N-(3-hydroxymethyl-phenyl)-amine

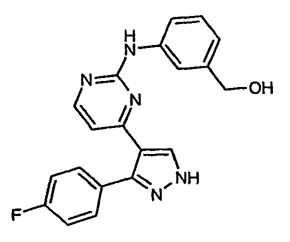

To a suspension of [4-(5-p-fluorphenyl-1H-pyrazol-4-yl)-pyrimidin-2-yl]-N-(3-methoxycarbonyl-phenyl)-amine (1.5g, 3.9mmol) in tetrahydrofuran (25ml) was added a solution of sodium dihydro-bis(2-methoxyethoxy)aluminate in toluene (6ml of a 3.5M solution) without cooling. The temperature of the reaction mixture rose to about +40°C and the starting material dissolved immediately yielding a yellow clear solution. After an additional hour the solution was poured onto crushed ice and ammonium chloride. The colorless precipitate was filtered and washed with ethyl acetate and tetrahydrofuran. The organic phases were combined, dried and concentrated under reduced pressure. Crystallization of the residue using diethylether gives the title compound, showing a m.p. of 221-223°C.

In an analogous manner to the above examples the compounds of the following tables may be obtained.
Table 01: Compounds of the general structure I.01, wherein each individual species corresponds to the combination of the definitions of R₄, R₅ and R₁₂ of the lines of table A. 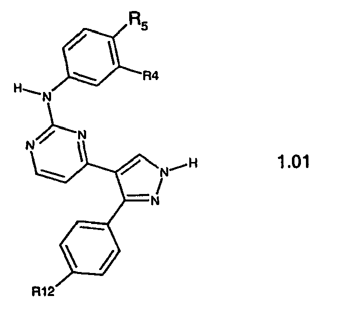 Table 02: Compounds of the general structure 1.02, wherein each individual species corresponds to the combination of the definitions R₄, R₅ and R₁₂ of the lines of table A. 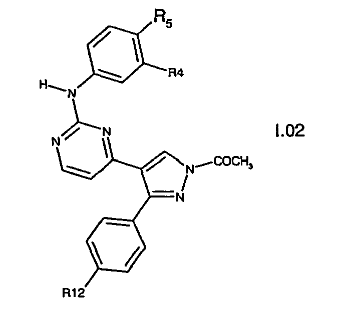 Table 03: Compounds of the general structure I.03, wherein each individual species corresponds to the combination of the definitions R₄, R₅ and R₁₂ of the lines of table A. 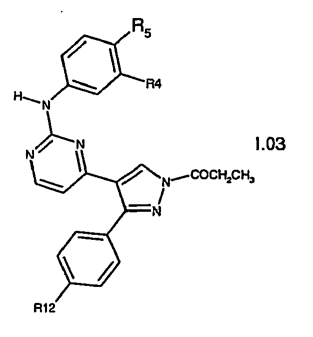 Table 04: Compounds of the general structure I.04, wherein each individual species corresponds to the combination of the definitions R₄, R₅ and R₁₂ of the lines of table A. 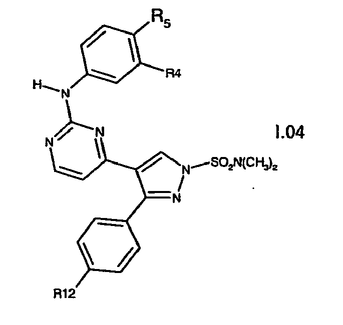 Table 05: Compounds of the general structure 1.05, wherein each individual species corresponds to the combination of the definitions R₄, R₅ and R₁₂ of the lines of table A. 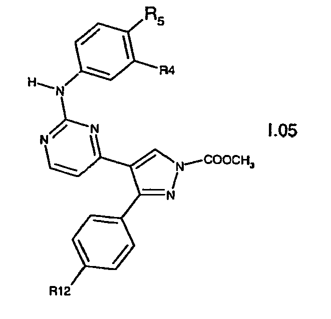 Table 06: Compounds of the general structure I.06, wherein each individual species corresponds to the combination of the definitions R₄ and R₁₂ of the lines of table A. 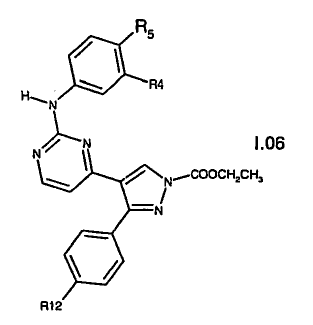 Table 07 : Compounds of the general structure 1.07, wherein each individual species corresponds to the combination of the definitions R₄, R₅ and R₁₂ of the lines of table A. 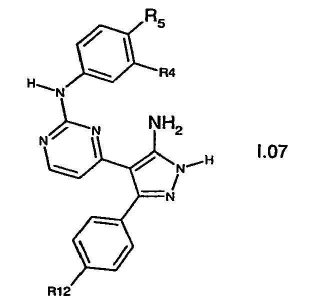 Table 08 : Compounds of the general structure 1.08, wherein each individual species corresponds to the combination of the definitions R₄, R₅ and R₁₂ of the lines of table A. 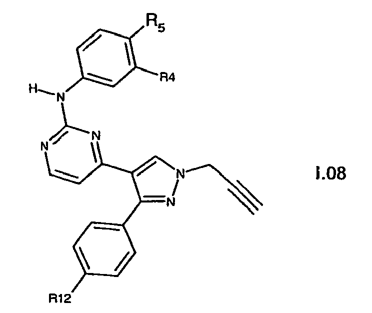 Table 09 : Compounds of the general structure 1.09, wherein each individual species corresponds to the combination of the definitions R₄, R₅ and R₁₂ of the lines of table A. 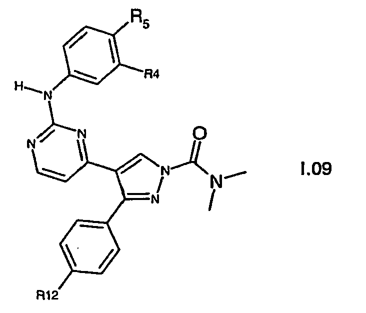 Table 10 : Compounds of the general structure 1.10, wherein each individual species corresponds to the combination of the definitions R₄, R₅ and R₁₂ of the lines of table A. 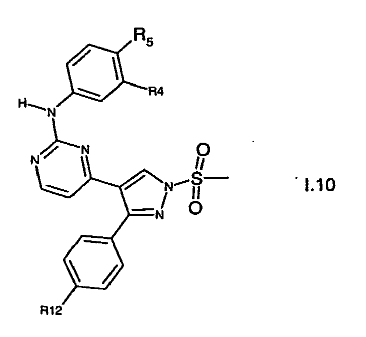 Table 11 : Compounds of the general structure I.11, wherein each individual species corresponds to the combination of the definitions R₄, R₅ and R₁₂ of the lines of table A. 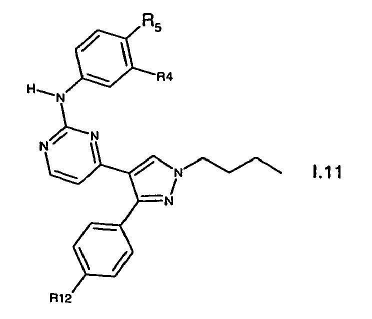 Table 12: Compounds of the general structure I.12, wherein each individual species corresponds to the combination of the definitions R₄, R₅ and R₁₂ of the lines of table A.

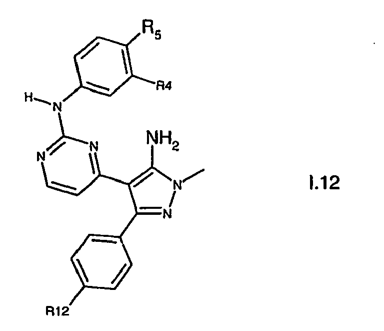

**Table A**

| Comp- No. | R₄ | R₁₂ | R₅ |
|---|---|---|---|
| 0001 | -CH₂-O-CH₃ | H | H |
| 0002 | -NH-CO-CH₃ | H | H |
| 0003 | -CH₂-NH-CO-CH₃ | H | H |
| 0004 | -CH(CH₃)-NH-CO-CH₃ | H | H |
| 0005 | -C(CH₃)₂-NH-CO-CH₃ | H | H |
| 0006 | -CH(CH₃)-O-CH₃ | H | H |
| 0007 | -C(CH₃)₂-O-CH₃ | H | H |
| 0008 | -CH(CH₃)-O-CO-CH₃ | H | H |
| 0009 | -CH₂-O-CO-CH₃ | H | H |
| 0010 | -C(CH₃)₂-O-CO-CH₃ | H | H |
| 0011 | -CH₂-CH₂-O-H | H | H |
| 0012 | -CH₂-CH₂-O-CH₃ | H | H |
| 0013 | -CH(CH₃)-CN | H | H |
| 0014 | -C(CH₃)₂-CN | H | H |
| 0015 | -CHz-CO-O-CH₃ | H | H |
| 0016 | 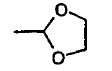 | H | H |
| 0017 | 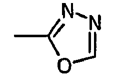 | H | H |
| 0018 | 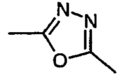 | H | H |
| 0019 | 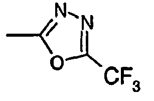 | H | H |
| 0020 | 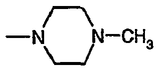 | H | H |
| 0021 | 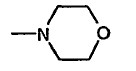 | H | H |
| 0022 | 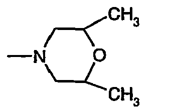 | H | H |
| 0023 | 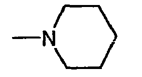 | H | H |
| 0024 | 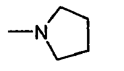 | H | H |
| 0025 | 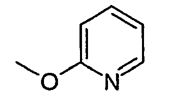 | H | H |
| 0026 | 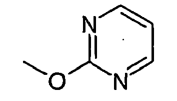 | H | H |
| 0027 | 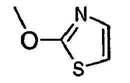 | H | H |
| 0028 | 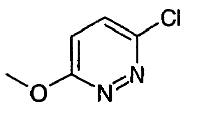 | H | H |
| 0029 | -O-CO-CH₃ | H | H |
| 0030 | -O-CO-CH₂-CH₃ | H | H |
| 0031 | -O-CO-N(CH₃)₂ | H | H |
| 0032 | -O-SO₂-N(CH₃)₂ | H | H |
| 0033 | -O-CO-O-CH₃ | H | H |
| 0034 | -O-CO-O-CH₂-CH₃ | H | H |
| 0035 | -CH₂-O-CH₃ | F | H |
| 0036 | -NH-CO-CH₃ | F | H |
| 0037 | -CH₂-NH-CO-CH₃ | F | H |
| 0038 | -CH(CH₃)-NH-CO-CH₃ | F | H |
| 0039 | -C(CH₃)₂-NH-CO-CH₃ | F | H |
| 0040 | -CH(CH₃)-O-CH₃ | F | H |
| 0041 | -C(CH₃)₂-O-CH₃ | F | H |
| 0042 | -CH(CH₃)-O-CO-CH₃ | F | H |
| 0043 | -CH₂-O-CO-CH₃ | F | H |
| 0044 | -C(CH₃)₂-O-CO-CH₃ | F | H |
| 0045 | -CH₂-CH₂-O-H | F | H |
| 0046 | -CH₂-CH₂-O-CH₃ | F | H |
| 0047 | -CH(CH₃)-CN | F | H |
| 0048 | -C(CH₃)₂-CN | F | H |
| 0049 | -CH₂-CO-O-CH₃ | F | H |
| 0050 | 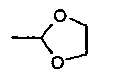 | F | H |
| 0051 | 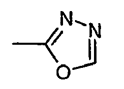 | F | H |
| 0052 | 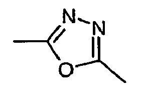 | F | H |
| 0053 | 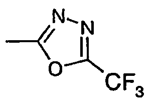 | F | H |
| 0054 | 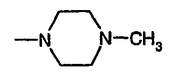 | F | H |
| 0055 | 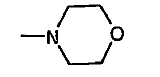 | F | H |
| 0056 | 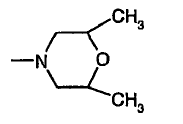 | F | H |
| 0057 | 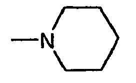 | F | H |
| 0058 | 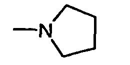 | F | H |
| 0059 | 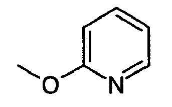 | F | H |
| 0060 | 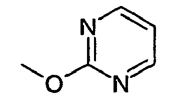 | F | H |
| 0061 | 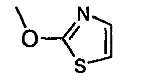 | F | H |
| 0062 | 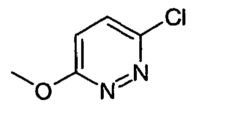 | F | H |
| 0063 | -O-CO-CH₃ | F | H |
| 0064 | -O-CO-CH₂-CH₃ | F | H |
| 0065 | -O-CO-N(CH₃)₂ | F | H |
| 0066 | -O-SO₂-N(CH₃)₂ | F | H |
| 0067 | -O-CO-O-CH₃ | F | H |
| 0068 | -O-CO-O-CH₂-CH₃ | Cl | H |
| 0069 | -CH₂-O-CH₃ | Cl | H |
| 0070 | -NH-CO-CH₃ | Cl | H |
| 0071 | -CH₂-NH-CO-CH₃ | Cl | H |
| 0072 | -CH(CH₃)-NH-CO-CH₃ | Cl | H |
| 0073 | -C(CH₃)₂-NH-CO-CH₃ | Cl | H |
| 0074 | -CH(CH₃)-O-CH₃ | Cl | H |
| 0075 | -C(CH₃)₂-O-CH₃ | Cl | H |
| 0076 | -CH(CH₃)-O-CO-CH₃ | Cl | H |
| 0077 | -CH₂-O-CO-CH₃ | Cl | H |
| 0078 | -C(CH₃)₂-O-CO-CH₃ | Cl | H |
| 0079 | -CH₂-CH₂-O-H | Cl | H |
| 0080 | -CH₂-CH₂-O-CH₃ | Cl | H |
| 0081 | -CH(CH₃)-CN | Cl | H |
| 0082 | -C(CH₃)₂-CN | Cl | H |
| 0083 | -CH₂-CO-O-CH₃ | Cl | H |
| 0084 | 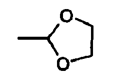 | Cl | H |
| 0085 | 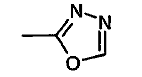 | Cl | H |
| 0086 | 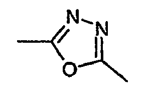 | Cl | H |
| 0087 | 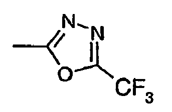 | Cl | H |
| 0088 | 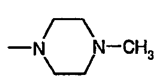 | Cl | H |
| 0089 | 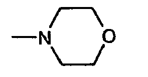 | Cl | H |
| 0090 | 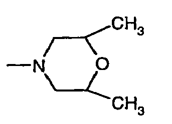 | Cl | H |
| 0091 | 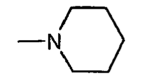 | Cl | H |
| 0092 | 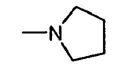 | Cl | H |
| 0093 | 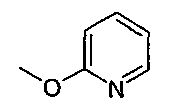 | Cl | H |
| 0094 | 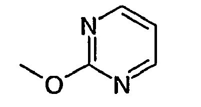 | Cl | H |
| 0095 | 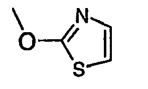 | Cl | H |
| 0096 | 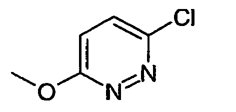 | Cl | H |
| 0097 | -O-CO-CH₃ | Cl | H |
| 0098 | -O-CO-CH₂-CH₃ | Cl | H |
| 0099 | -O-CO-N(CH₃)₂ | Cl | H |
| 0100 | -O-SO₂-N(CH₃)₂ | Cl | H |
| 0101 | -O-CO-O-CH₃ | Cl | H |
| 0102 | -O-CO-O-CH₂-CH₃ | Cl | H |
| 0103 | -CH₂-O-CH₃ | Br | H |
| 0104 | -NH-CO-CH₃ | Br | H |
| 0105 | -CH₂-NH-CO-CH₃ | Br | H |
| 0106 | -CH(CH₃)-NH-CO-CH₃ | Br | H |
| 0107 | -C(CH₃)₂-NH-CO-CH₃ | Br | H |
| 0108 | -CH(CH₃)-O-CH₃ | Br | H |
| 0109 | -C(CH₃)₂-O-CH₃ | Br | H |
| 0110 | -CH(CH₃)-O-CO-CH₃ | Br | H |
| 0111 | -CH₂-O-CO-CH₃ | Br | H |
| 0112 | -C(CH₃)₂-O-CO-CH₃ | Br | H |
| 0113 | -CH₂-CH₂-O-H | Br | H |
| 0114 | -CH₂-CH₂-O-CH₃ | Br | H |
| 0115 | -CH(CH₃)-CN | Br | H |
| 0116 | -C(CH₃)₂-CN | Br | H |
| 0117 | -CH₂-CO-O-CH₃ | Br | H |
| 0118 | 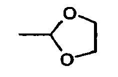 | Br | H |
| 0119 | 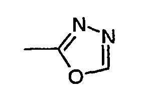 | Br | H |
| 0120 | 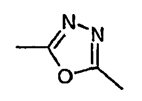 | Br | H |
| 0121 | 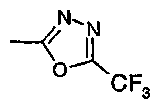 | Br | H |
| 0122 | 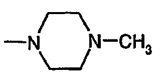 | Br | H |
| 0123 | 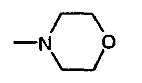 | Br | H |
| 0124 | 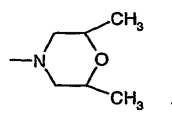 | Br | H |
| 0125 | 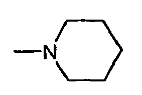 | Br | H |
| 0126 | 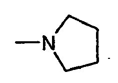 | Br | H |
| 0127 | 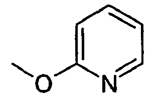 | Br | H |
| 0128 | 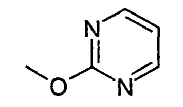 | Br | H |
| 0129 | 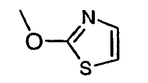 | Br | H |
| 0130 | 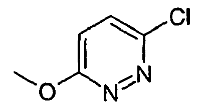 | Br | H |
| 0131 | -O-CO-CH₃ | Br | H |
| 0132 | -O-CO-CH₂-CH₃ | Br | H |
| 0133 | -O-CO-N(CH₃)₂ | Br | H |
| 0134 | -O-SO₂-N(CH₃)₂ | Br | H |
| 0135 | -O-CO-O-CH₃ | Br | H |
| 0136 | -O-CO-O-CH₂-CH₃ | Br | H |
| 0137 | -CH₂-O-CH₃ | CH₃ | H |
| 0138 | -NH-CO-CH₃ | CH₃ | H |
| 0139 | -CH₂-NH-CO-CH₃ | CH₃ | H |
| 0140 | -CH(CH₃)-NH-CO-CH₃ | CH₃ | H |
| 0141 | -C(CH₃)₂-NH-CO-CH₃ | CH₃ | H |
| 0142 | -CH(CH₃)-O-CH₃ | CH₃ | H |
| 0143 | -C(CH₃)₂-O-CH₃ | CH₃ | H |
| 0144 | -CH(CH₃)-O-CO-CH₃ | CH₃ | H |
| 0145 | -CH₂-O-CO-CH₃ | CH₃ | H |
| 0146 | -C(CH₃)₂-O-CO-CH₃ | CH₃ | H |
| 0147 | -CH₂-CH₂-O-H | CH₃ | H |
| 0148 | -CH₂-CH₂-O-CH₃ | CH₃ | H |
| 0149 | -CH(CH₃)-CN | CH₃ | H |
| 0150 | -C(CH₃)₂-CN | CH₃ | H |
| 0151 | -CH₂-CO-O-CH₃ | CH₃ | H |
| 0152 | 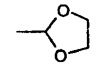 | CH₃ | H |
| 0153 | 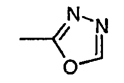 | CH₃ | H |
| 0154 | 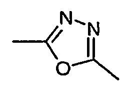 | CH₃ | H |
| 0155 | 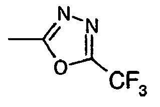 | CH₃ | H |
| 0156 | 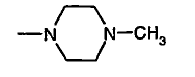 | CH₃ | H |
| 0157 | 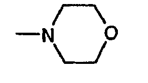 | CH₃ | H |
| 0158 | 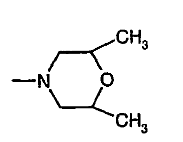 | CH₃ | H |
| 0159 | 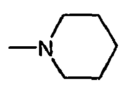 | CH₃ | H |
| 0160 | 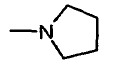 | CH₃ | H |
| 0161 | 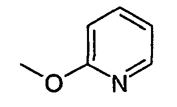 | CH₃ | H |
| 0162 | 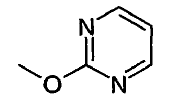 | CH₃ | H |
| 0163 | 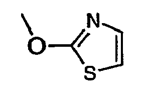 | CH₃ | H |
| 0164 | 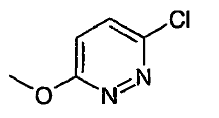 | CH₃ | H |
| 0165 | -O-CO-CH₃ | CH₃ | H |
| 0166 | -O-CO-CH₂-CH₃ | CH₃ | H |
| 0167 | -O-CO-N(CH₃)₂ | CH₃ | H |
| 0168 | -O-SO₂-N(CH₃)₂ | CH₃ | H |
| 0169 | -O-CO-O-CH₃ | CH₃ | H |
| 0170 | -O-CO-O-CH₂-CH₃ | CH₃ | H |
| 0171 | -CH₂-O-CH₃ | OCH₃ | H |
| 0172 | -NH-CO-CH₃ | OCH₃ | H |
| 0173 | -CH₂-NH-CO-CH₃ | OCH₃ | H |
| 0174 | -CH(CH₃)-NH-CO-CH₃ | OCH₃ | H |
| 0175 | -C(CH₃)₂-NH-CO-CH₃ | OCH₃ | H |
| 0176 | -CH(CH₃)-O-CH₃ | OCH₃ | H |
| 0177 | -C(CH₃)₂-O-CH₃ | OCH₃ | H |
| 0178 | -CH(CH₃)-O-CO-CH₃ | OCH₃ | H |
| 0179 | -CH₂-O-CO-CH₃ | OCH₃ | H |
| 0180 | -C(CH₃)₂-O-CO-CH₃ | OCH₃ | H |
| 0181 | -CH₂-CH₂-O-H | OCH₃ | H |
| 0182 | -CH₂-CH₂-O-CH₃ | OCH₃ | H |
| 0183 | -CH(CH₃)-CN | OCH₃ | H |
| 0184 | -C(CH₃)₂-CN | OCH₃ | H |
| 0185 | -CH₂-CO-O-CH₃ | OCH₃ | H |
| 0186 | 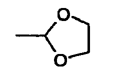 | OCH₃ | H |
| 0187 | 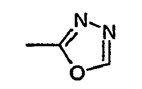 | OCH₃ | H |
| 0188 | 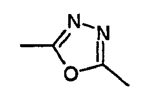 | OCH₃ | H |
| 0189 | 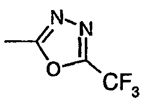 | OCH₃ | H |
| 0190 | 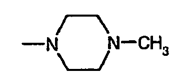 | OCH₃ | H |
| 0191 | 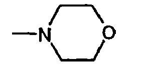 | OCH₃ | H |
| 0192 | 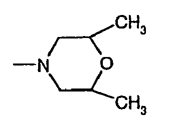 | OCH₃ | H |
| 0193 | 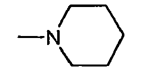 | OCH₃ | H |
| 0194 | 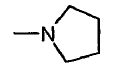 | OCH₃ | H |
| 0195 | 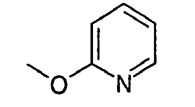 | OCH₃ | H |
| 0196 | 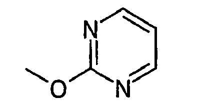 | OCH₃ | H |
| 0197 | 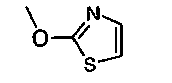 | OCH₃ | H |
| 0198 | 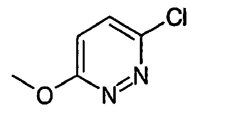 | OCH₃ | H |
| 0199 | -O-CO-CH₃ | OCH₃ | H |
| 0200 | -O-CO-CH₂-CH₃ | OCH₃ | H |
| 0201 | -O-CO-N(CH₃)₂ | OCH₃ | H |
| 0202 | -O-SO₂-N(CH₃)₂ | OCH₃ | H |
| 0203 | -O-CO-O-CH₃ | OCH₃ | H |
| 0204 | -O-CO-O-CH₂-CH₃ | OCH₃ | H |
| 0205 | -CH₂-O-CH₃ | OC₂H₅ | H |
| 0206 | -NH-CO-CH₃ | OC₂H₅ | H |
| 0207 | -CH₂-NH-CO-CH₃ | OC₂H₅ | H |
| 0208 | -CH(CH₃)-NH-CO-CH₃ | OC₂H₅ | H |
| 0209 | -C(CH₃)₂-NH-CO-CH₃ | OC₂H₅ | H |
| 0210 | -CH(CH₃)-O-CH₃ | OC₂H₅ | H |
| 0211 | -C(CH₃)₂-O-CH₃ | OC₂H₅ | H |
| 0212 | -CH(CH₃)-O-CO-CH₃ | OC₂H₅ | H |
| 0213 | -CH₂-O-CO-CH₃ | OC₂H₅ | H |
| 0214 | -C(CH₃)₂-O-CO-CH₃ | OC₂H₅ | H |
| 0215 | -CH₂-CH₂-O-H | OC₂H₅ | H |
| 0216 | -CH₂-CH₂-O-CH₃ | OC₂H₅ | H |
| 0217 | -CH(CH₃)-CN | OC₂H₅ | H |
| 0218 | -C(CH₃)₂-CN | OC₂H₅ | H |
| 0219 | -CH₂-CO-O-CH₃ | OC₂H₅ | H |
| 0220 | 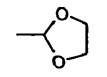 | OC₂H₅ | H |
| 0221 | 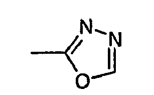 | OC₂H₅ | H |
| 0222 | 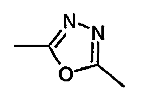 | OC₂H₅ | H |
| 0223 | 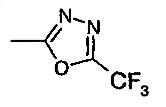 | OC₂H₅ | H |
| 0224 | 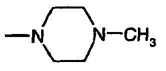 | OC₂H₅ | H |
| 0225 | 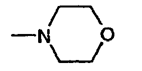 | OC₂H₅ | H |
| 0226 | 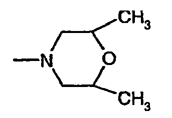 | OC₂H₅ | H |
| 0227 | 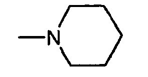 | OC₂H₅ | H |
| 0228 | 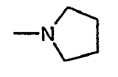 | OC₂H₅ | H |
| 0229 | 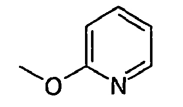 | OC₂H₅ | H |
| 0230 | 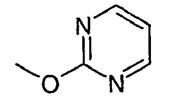 | OC₂H₅ | H |
| 0231 | 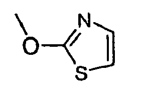 | OC₂H₅ | H |
| 0232 | 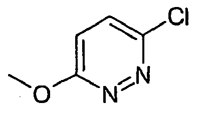 | OC₂H₅ | H |
| 0233 | -O-CO-CH₃ | OC₂H₅ | H |
| 0234 | -O-CO-CH₂-CH₃ | OC₂H₅ | H |
| 0235 | -O-CO-N(CH₃)₂ | OC₂H₅ | H |
| 0236 | -O-SO₂-N(CH₃)₂ | OC₂H₅ | H |
| 0237 | -O-CO-O-CH₃ | OC₂H₅ | H |
| 0238 | -O-CO-O-CH₂-CH₃ | OC₂H₅ | H |
| 0239 | -CH₂-O-CH₃ | C₂H₅ | H |
| 0240 | -NH-CO-CH₃ | C₂H₅ | H |
| 0241 | -CH₂-NH-CO-CH₃ | C₂H₅ | H |
| 0242 | -CH(CH₃)-NH-CO-CH₃ | C₂H₅ | H |
| 0243 | -C(CH₃)₂-NH-CO-CH₃ | C₂H₅ | H |
| 0244 | -CH(CH₃)-O-CH₃ | C₂H₅ | H |
| 0245 | -C(CH₃)₂-O-CH₃ | C₂H₅ | H |
| 0246 | -CH(CH₃)-O-CO-CH₃ | C₂H₅ | H |
| 0247 | -CH₂-O-CO-CH₃ | C₂H₅ | H |
| 0248 | -C(CH₃)₂-O-CO-CH₃ | C₂H₅ | H |
| 0249 | -CH₂-CH₂-O-H | C₂Hₛ | H |
| 0250 | -CH₂-CH₂-O-CH₃ | C₂H₅ | H |
| 0251 | -CH(CH₃)-CN | C₂H₅ | H |
| 0252 | -C(CH₃)₂-CN | C₂H₅ | H |
| 0253 | -CH₂-CO-O-CH₃ | C₂H₅ | H |
| 0254 | 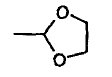 | C₂H₅ | H |
| 0255 | 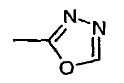 | C₂H₅ | H |
| 0256 | 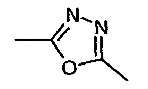 | C₂H₅ | H |
| 0257 | 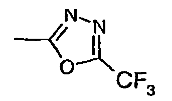 | C₂H₅ | H |
| 0258 | 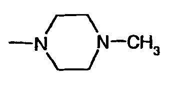 | C₂H₅ | H |
| 0259 | 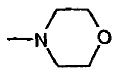 | C₂H₅ | H |
| 0260 | 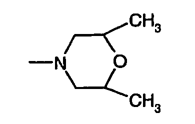 | C₂H₅ | H |
| 0261 | 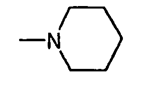 | C₂H₅ | H |
| 0262 | 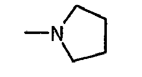 | C₂H₅ | H |
| 0263 | 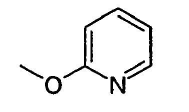 | C₂H₅ | H |
| 0264 | 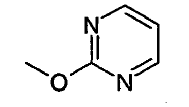 | C₂H₅ | H |
| 0265 | 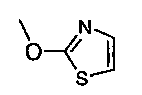 | C₂H₅ | H |
| 0266 | 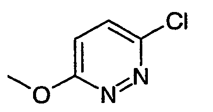 | C₂H₅ | H |
| 0267 | -O-CO-CH₃ | C₂H₅ | H |
| 0268 | -O-CO-CH₂-CH₃ | C₂H₅ | H |
| 0269 | -O-CO-N(CH₃)₂ | C₂H₅ | H |
| 0270 | -O-SO₂-N(CH₃)₂ | C₂H₅ | H |
| 0271 | -O-CO-O-CH₃ | C₂H₅ | H |
| 0272 | -O-CO-O-CH₂-CH₃ | C₂H₅ | H |
| 0273 | H | H | H |
| 0274 | H | F | H |
| 0275 | H | Cl | H |
| 0276 | H | Br | H |
| 0277 | H | CH₃ | H |
| 0278 | H | OCH₃ | H |
| 0279 | H | OC₂H₅ | H |
| 0280 | H | C₂H₅ | H |
| 0281 | CN | H | H |
| 0282 | CN | F | H |
| 0283 | CN | Cl | H |
| 0284 | CN | Br | H |
| 0285 | CN | CH₃ | H |
| 0286 | CN | OCH₃ | H |
| 0287 | CN | OC₂H₅ | H |
| 0288 | CN | C₂H₅ | H |
| 0289 | OH | H | H |
| 0290 | OH | F | H |
| 0291 | OH | Cl | H |
| 0292 | OH | Br | H |
| 0293 | OH | CH₃ | H |
| 0294 | OH | OCH₃ | H |
| 0295 | OH | OC₂H₅ | H |
| 0296 | OH | C₂H₅ | H |
| 0297 | CH₂CN | H | H |
| 0298 | CH₂CN | F | H |
| 0299 | CH₂CN | Cl | H |
| 0300 | CH₂CN | Br | H |
| 0301 | CH₂CN | CH₃ | H |
| 0302 | CH₂CN | OCH₃ | H |
| 0303 | CH₂CN | OC₂H₅ | H |
| 0304 | CH₂CN | C₂H₅ | H |
| 0305 | OCH₂CN | H | H |
| 0306 | OCH₂CN | F | H |
| 0307 | OCH₂CN | Cl | H |
| 0308 | OCH₂CN | Br | H |
| 0309 | OCH₂CN | CH₃ | H |
| 0310 | OCH₂CN | OCH₃ | H |
| 0311 | OCH₂CN | OC₂H₅ | H |
| 0312 | OCH₂CN | C₂H₅ | H |
| 0313 | COOCH₃ | H | H |
| 0314 | COOCH₃ | F | H |
| 0315 | COOCH₃ | Cl | H |
| 0316 | COOCH₃ | Br | H |
| 0317 | COOCH₃ | CH₃ | H |
| 0318 | COOCH₃ | OCH₃ | H |
| 0319 | COOCH₃ | OC₂H₅ | H |
| 0320 | COOCH₃ | C₂H₅ | H |
| 0321 | CH₂O | H | H |
| 0322 | CH₂O | F | H |
| 0323 | CH₂OH | Cl | H |
| 0324 | CH₂O | Br | H |
| 0325 | CH₂O | CH₃ | H |
| 0326 | CH₂OH | OCH₃ | H |
| 0327 | CH₂OH | OC₂H₅ | H |
| 0328 | CH₂OH | C₂H₅ | H |
| 0329 | COCH₃ | H | H |
| 0330 | COCH₃ | F | H |
| 0331 | COCH₃ | Cl | H |
| 0332 | COCH₃ | Br | H |
| 0333 | COCH₃ | CH₃ | H |
| 0334 | COCH₃ | OCH₃ | H |
| 0335 | COCH₃ | OC₂H₅ | H |
| 0336 | COCH₃ | C₂H₅ | H |
| 0337 | CH(OH)CH₃ | H | H |
| 0338 | CH(OH)CH₃ | F | H |
| 0339 | CH(OH)CH₃ | Cl | H |
| 0340 | CH(OH)CH₃ | Br | H |
| 0341 | CH(OH)CH₃ | CH₃ | H |
| 0342 | CH(OH)CH₃ | OCH₃ | H |
| 0343 | CH(OH)CH₃ | OC₂H₅ | H |
| 0344 | CH(OH)CH₃ | C₂H₅ | H |
| 0345 | C(CH₃)₂CH₂O | H | H |
| 0346 | C(CH₃)₂CH₂O | F | H |
| 0347 | C(CH₃)₂CH₂OH | Cl | H |
| 0348 | C(CH₃)₂CH₂OH | Br | H |
| 0349 | C(CH₃)₂CH₂O | CH₃ | H |
| 0350 | C(CH₃)₂CH₂OH | OCH₃ | H |
| 0351 | C(CH₃)₂CH₂OH | OC₂H₅ | H |
| 0352 | C(CH₃)₂CH₂OH | C₂H₅ | H |
| 0353 | C(CH₃)₂O | H | H |
| 0354 | C(CH₃)₂OH | F | H |
| 0355 | C(CH₃)₂OH | Cl | H |
| 0356 | C(CH₃)₂OH | CH₃ | H |
| 0357 | C(CH₃, CH₂CH₃)OH | H | H |
| 0358 | C(CH₃, CH₂CH₃)OH | F | H |
| 0359 | C(CH3, CH2CH3)OH | Cl | H |
| 0360 | C(CH₃, CH₂CH₃)OH | CH₃ | H |
| 0361 | C(CH₃, CH₂CH=CH₂)OH | H | H |
| 0362 | C(CH₃, CH₂CH=CH₂)OH | F | H |
| 0363 | C(CH₃, CH₂CH=CH₂)OH | Cl | H |
| 0364 | C(CH₃, CH₂CH=CH₂)OH | CH₃ | H |
| 0365 | CONHCH₃ | H | H |
| 0366 | CONHCH₃ | F | H |
| 0367 | CONHCH₃ | Cl | H |
| 0368 | CONHCH₃ | CH₃ | H |
| 0369 | CON(CH₃)₂ | H | H |
| 0370 | CON(CH₃)₂ | F | H |
| 0371 | CON(CH₃)₂ | Cl | H |
| 0372 | CON(CH₃)₂ | CH₃ | H |
| 0373 | C(CH₃)=NOCH₃ | H | H |
| 0374 | C(CH₃)=NOCH₃ | F | H |
| 0375 | C(CH₃)=NOCH₃ | Cl | H |
| 0376 | C(CH₃)=NOCH₃ | CH₃ | H |
| 0377 | C(CH₃)=OH | H | H |
| 0378 | C(CH₃)=NOH | F | H |
| 0379 | C(CH₃)=NOH | Cl | H |
| 0380 | C(CH₃)=NOH | CH₃ | H |
| 0381 | OCH₂C≡CH | H | H |
| 0382 | OCH₂C≡CH | F | H |
| 0383 | OCH₂C≡CH | Cl | H |
| 0384 | OCH₂C≡CH | CH₃ | H |
| 0385 | OCH₂OCH₂CH₃ | H | H |
| 0386 | OCH₂OCH₂CH₃ | F | H |
| 0387 | OCH₂OCH₂CH | Cl | H |
| 0388 | OCH₂OCH₂CH₃ | CH₃ | H |
| 0389 | OCH₃ | H | H |
| 0390 | OCH₃ | F | H |
| 0391 | OCH₃ | Cl | H |
| 0392 | OCH₃ | CH₃ | H |
| 0393 | Cl | H | H |
| 0394 | Cl | F | H |
| 0395 | Cl | Cl | H |
| 0396 | Cl | CH₃ | H |
| 0397 | F | H | H |
| 0398 | F | F | H |
| 0399 | F | Cl | H |
| 0400 | F | CH₃ | H |
| 0401 | H | H | F |
| 0402 | H | F | F |
| 0403 | H | Cl | F |
| 0404 | H | CH₃ | F |
| 0405 | H | H | Cl |
| 0406 | H | F | Cl |
| 0407 | H | Cl | Cl |
| 0408 | H | CH₃ | Cl |
| 0409 | OCH₂CH=CH₂ | H | H |
| 0410 | OCH₂CH=CH₂ | F | H |
| 0411 | OCH₂CH=CH₂ | CI | H |
| 0412 | OCH₂CH=CH₂ | CH₃ | H |
| 413 | OCH₂CH₂CH₃ | H | H |
| 414 | OCH₂CH₂CH₃ | F | H |
| 415 | OCH₂CH₂CH₃ | Cl | H |
| 416 | OCH₂CH₂CH₃ | CH₃ | H |

For the following example compounds physico-chemical data have been obtained and are displayed in order to illustrate the working of the present invention, including the outlined methods of synthesis. The number of given data may not be interpreted as a limitation of the invention.

**Table B:**

| Comp. No. | Melting point [°C] or ¹H-NMR [δ in ppm] | | Comp. No. | Melting point [°C] or ¹H-NMR [δ in ppm] | | Comp. No. | Melting point [°C] or ¹H-NMR [δ in ppm] |
|---|---|---|---|---|---|---|---|
| 01.0008 | 199-200 | | 02.0008 | resinous | | 07.0329 | |
| 01.0273 | > 260 | | 02.0033 | 147-148 | | 07.0330 | |
| 01.0274 | > 260 | | 02.0043 | 133-134 | | 07.0330 | |
| 01.0281 | > 260 | | 02.0329 | 192-193 | | 07.0331 | |
| 01.0289 | 233-235 | | 03.0034 | 141-142 | | 07.0382 | |
| 01.0297 | 230-231 | | 03.0329 | 188 | | 07.0383 | |
| 01.0313 | > 250 | | 04.0032 | 168-169 | | 07.0385 | |
| 01.0314 | > 265 | | 04.0313 | 196 | | 07.0386 | |
| 01.0322 | 221-223 | | 04.0329 | 178-179 | | 07.0387 | |
| 01.0329 | >260 | | 05.0329 | 182-183 | | 07.0409 | |
| 01.0330 | 253-254 | | 07.0329 | 249-250 | | 07.0410 | |
| 01.0337 | 227-228 | | 07.0389 | 252-253 | | 07.0411 | |
| 01.0353 | 263-264 | | 07.0393 | 250-251 | | 07.0413 | |
| 01.0354 | 235-236 | | 07.0394 | 257-258 | | 07.0414 | |
| 01.0357 | 236-237 | | 07.0397 | 243-244 | | 07.0415 | |
| 01.0361 | 208-210 | | 07.0401 | 256-258 | | 07.3810 | |
| 01.0365 | 182-183 | | 07.0405 | 291-292 | | 12.0329 | |
| 01.0369 | 289-290 | | 08.0289 | 191-195 | | 12.0330 | |
| 01.0373 | >220 | | 08.0385 | resinous | | 12.0389 | |
| 01.0377 | >220 | | 09.0313 | 181 | | 12.0390 | |
| 01.0381 | >200 | | 09.0329 | 198-199 | | | |
| 01.0385 | 205-207 | | 10.0329 | 189-190 | | | |
| | | | 11.0289 | solid | | | |
| | | | 11.0385 | resinous | | | |
| | | | | | | | |

In the following, examples of test systems in plant protection are provided which can demonstrate the efficiency of the compounds of the formula I (designated as "active ingredient"or "test compounds"):

### Biological Examples

### Example B-1: Effect against Puccinia graminis on wheat (brownrust on wheat)

### a) Residual protective activity

1 week old wheat plants cv. Arina are treated with the formulated test-compound (0.02 % active substance) in a spray chamber. Two days after application wheat plants are inoculated by spraying a spore suspension (1 x 10⁵ ureidospores/ml) on the test plants. After an incubation period of 1 day at +20°C and 95% relative atmospheric humidity (r. h.) plants are kept for 9 days at +20°C and 60% r.h. in a greenhouse. The disease incidence is assessed 10 days after inoculation.

### b) Systemic activity

An aqueous spray liquor prepared from the formulated test compound (0.002 % active substance, based on the volume of soil) is poured onto wheat plants 5 days after sowing. Care is taken that the spray liquor does not come into contact with the above-ground parts of the plant. 48 hours later, the plants are inoculated with a spore suspension of the fungus. After an incubation period of 48 hours (95 to 100 % r.h. at +20°C), the plants are placed in a greenhouse at +20°C. 12 days after infection, the disease incidence is evaluated.

### Example B-2: Effect against Phytophthora infestans on tomatoes (late blight on potato)

### a) Residual protective activity

3 week old tomato plants cv. Roter Gnom are treated with the formulated test compound (0.02 % active substance) in a spray chamber. Two day after application the plants are inoculated by spraying a sporangia suspension (2 x 10⁴ sporangia/ml) on the test plants. After an incubation period of 4 days at +18°C and 95% r. h. in a growth chamber the disease incidence is assessed.

### b) Systemic activity

An aqueous suspension prepared from the formulated test compound (0.002 % active substance, based on the volume of soil) is poured onto tomato plants which have been cultivated for three weeks. Care is taken that the spray liquor does not come into contact with the above-ground parts of the plant. 48 hours later, the plants are inoculated with a sporangia suspension of the fungus. Evaluation of the disease incidence takes place 5 days after infection, during which period conditions of 90 to 100 % r.h. and +20°C are maintained.

### Example B-3: Effect against Phytophthora infestans / potato (late blight on potato)

5 week old potato plants cv. Bintje are treated with the formulated test compound (0.02 % active substance) in a spray chamber. Two days after application the plants are inoculated by spraying a sporangia suspension (1.4 x 10⁵ sporangia/ml) on the test plants. After an incubation period of 4 days at +18°C and 95% r. h. in a growth chamber the disease incidence is assessed.

### Example B-4: Effect against Plasmopara viticola on grapevine (grape downy mildew)

5 week old grape seedlings cv. Gutedel are treated with the formulated test compound (0.02 % active substance) in a spray chamber. One day after application grape plants are inoculated by spraying a sporangia suspension (4 x 10⁴ sporangia/ml) on the lower leaf side of the test plants. After an incubation period of 6 days at +22°C and 95% r. h. in a greenhouse the disease incidence is assessed.

### Example B-5: Residual protective activity against Venturia inaegualis on apples (scab on apple)

4 week old apple seedlings cv. McIntosh are treated with the formulated test compound (0.02 % active substance) in a spray chamber. One day after application apple plants are inoculated by spraying a spore suspension (4 x 10⁵ conidia/ml) on the test plants. After an incubation period of 4 days at +21°C and 95% r. h. the plants are placed for 4 days at +21°C and 60% r. h. in a greenhouse. After another 4 day incubation period at +21°C and 95% r. h. the disease incidence is assessed.

### Example B-6: Effect against Erysiphe graminis on barley (powdery mildew on barley)

### a) Residual protective activity

Barley plants of approximately 8 cm height are sprayed to drip point with an aqueous spray liquor prepared from wettable powder of the active ingredient (0.02 % active substance), and dusted 3 to 4 hours later with conidia of the fungus. The infected plants are placed in a greenhouse at +22°C. 12 days after infection, the fungal attack is evaluated.

### b) Systemic activity

An aqueous spray liquor prepared from the formulated test compound (0.002 % active substance, based on the volume of soil) is poured onto barley plants of approximately 8 cm height. Care is taken that the spray liquor does not come into contact with the above-ground parts of the plant. 48 hours later, the plants are dusted with conidia of the fungus. The infected plants are placed in a greenhouse at +22°C. 12 days after infection, the disease incidence is evaluated.

### Example B-7: Botrytis cinerea /grape (botrytis on grapes)

5 week old grape seedlings cv. Gutedel are treated with the formulated test compound (0.02% active substance) in a spray chamber. Two days after application grape plants are inoculated by spraying a spore suspension (1 x 10⁶ conidia/ml) on the test plants. After an incubation period of 4 days at +21°C and 95% r. h. in a greenhouse the disease incidence is assessed.

### Example B-8: Effect against Botrytis cinerea l tomato (botrytis on tomatoes)

4 week old tomato plants cv. Roter Gnom are treated with the formulated test compound 0.02 % active substance) in a spray chamber. Two days after application tomato plants are inoculated by spraying a spore suspension (1 x 10⁵ conidia/ml) on the test plants. After an incubation period of 4 days at +20°C and 95% r. h. in a greenhouse the disease incidence is assessed.

### Example B-9: Effect against Pyricularia oryzae / rice (rice blast)

3 week old rice plants cv. Sasanishiki are treated with the formulated test compound (0.02 % active substance) in a spray chamber. Two days after application rice plants are inoculated by spraying a spore suspension (1 x 10⁵ conidia/ml) on the test plants. After an incubation period of 6 days at +25°C and 95% r. h. the disease incidence is assessed.

### Example B-10: Effect against Pyrenophora teres (Helminthosporium) / barley (net blotch on barley)

1 week old barley plants cv. Regina are treated with a formulated test compound (0.02 % active substance) in a spray chamber. Two days after application barley plants are inoculated by spraying a spore suspension (3 x 10⁴ conidia/ml) on the test plants. After an incubation period of 2 days at +20°C and 95% r.h. plants are kept for 2 days at +20°C and 60% r.h. in a greenhouse. The disease incidence is assessed 4 days after inoculation.

### Example B-11: Effect against Fusarium culmorum/wheat (fusarium head blight on wheat)

A conidia suspension of F. *culmorum* (7 x 10⁵ conidia/ml) is mixed with the formulated test compound (0.002 % active substance).. The mixture is applied into a pouch which has been equipped before with a filter paper. After the application wheat seeds (cv. Orestis) are sown into the upper fault of the filter paper. The prepared pouches are then incubated for 11 days at approx. +10°C to +18°C and a relative humidity of 100% with a light period of 14 hours. The evaluation is made by assessing the degree of disease occurrence in the form of brown lesions on the roots.

### Example B-12: Effect against Septoria nodorum/wheat (septoria leaf spot on wheat)

1 week old wheat plants cv. Arina are treated with a formulated test compound (0.02 % active substance) in a spray chamber. One day after application wheat plants are inoculated by spraying a spore suspension (5 x 10⁵ conidia/ml) on the test plants. After an incubation period of 1 day at +20°C and 95% r.h. plants are kept for 10 days at +20°C and 60% r.h. in a greenhouse. The disease incidence is assessed 11 days after inoculation.

## Claims

1. A compound of the formula I 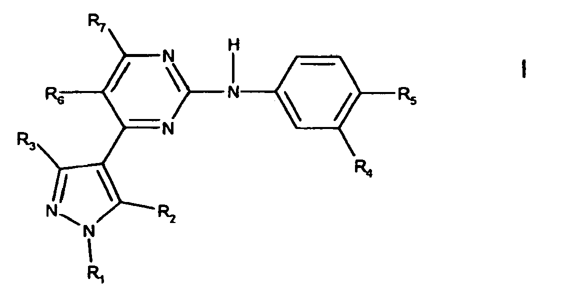 wherein
R₁ is hydrogen, COCH₃, COCH₂CH₃, SO₂N(CH₃)₂, COOCH₃, COOCH₂CH₃, CON(CH₃)₂, SO₂CH₃, CH₂C≡CH, methyl, ethyl, n-propyl or n-butyl;
R₂ is hydrogen or amino;
R₃ is phenyl or thienyl either of which may be optionally substituted by one, two or three substituents R₁₂ wherein each of the R₁₂ groups may be the same or different;
R₄ is hydrogen, hydroxy, cyano, fluorine, chlorine, bromine, 2-hydroximino-ethyl, 2-methoximino-ethyl, C₁-C₄alkyl, C₁-C₄alkoxy, C₁-C₄haloalkoxy, C₁-C₄haloalkyl, C₁-C₄cyanoalkyl, C₁-C₄alkanoyloxy, C₁-C₄hydroxyalkyl, C₁-C₄haloalkanoyloxy, C₁-C₄alkanoyl-C₁-C₆aminoalkyl, C₁-C₄alkanoyloxy-C₁-C₄alkyl, C₁-C₄alkanoyl, C₁-C₄alkylthio, C₁-C₄alkoxycarbonyl, heteroaryl or heterocyclyl, C₁-C₄alkanoyloxy, C₁-C₄alkyloxycarbonyloxy, C₁₋C₄alkanoyloxy, di(C₁-C₄alkyl)aminoC₁-C₄alkanoyloxy or di(C₁-C₄alkyl)aminosulfonyloxy R₅ is hydrogen, C₁-C₃ alkyl, hydroxy, C₁-C₃ alkoxy or halogen;
R₆ is hydrogen, C₁-C₃ alkyl, halogen or C₁-C₃ alkoxy;
R₇ is hydrogen, C₁-C₃ alkyl, halogen or C₁-C₃ alkoxy;
R₁₂ is halogen, C₁-C₄ alkyl or C₁-C₄ alkoxy.

2. A compound according to claim 1, which is a compound of formula IA 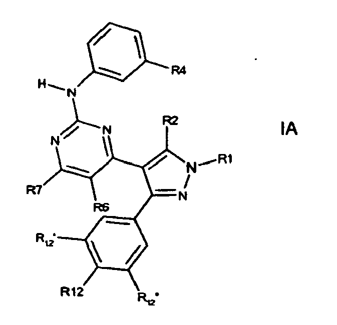 wherein R₁, R₂, R₄, R₆ and R₇ are as defined in relation to Formula I in claim 1 and R₁₂, R_{12'} and R_{12''} are independently as defined for R₁₂ in formula I.

3. A compound according to claim 2 wherein R₆, R₇, R_{12'} and R_{12''} are all hydrogen.

4. A compound according to any preceding claim where R₄ is hydrogen, hydroxy, cyano, fluorine, chlorine, bromine, methyl, tert. butyl, methylthio, trifluoromethyl, cyanomethyl, 2-cyanoethyl, hydroxymethyl, 1-hydroxyethyl, 2-hydroxyisopropyl, acetyl, 2-hydroximino-ethyl, 2-methoximino-ethyl, acetoxymethyl, methoxycarbonyl, methoxy, ethoxy or trifluoromethoxy oxadiazolyl (optionally substituted by methyl or trifluoromethyl), piperidinyl, morpholinyl, piperazinyl, pyridyloxy, pyrimidinyloxy, thiazolyloxy, pyridazinyloxy, C₁-C₄alkanoyloxy, C₁-C₄alkyloxycarbonyloxy, C₁-C₄alkanoyloxy, di(C₁₋C₄alkyl)aminoC₁-C₄alkanoyloxy or di(C₁-C₄alkyl)aminosulfonyloxy,

5. A compound according to any preceding claim where R₁₂ is H or is fluoro, chloro, bromo, methyl, ethyl, methoxy or ethoxy.

6. A composition for controlling and protecting against phytopathogenic microorganisms, comprising a compound of formula I according to claim 1 as active ingredient together with a suitable carrier or diluent.

7. The use of a compound of formula I according to claim 1 in protecting plants against infestation by phytopathogenic microorganisms.

8. A method of controlling and preventing an infestation of crop plants by phytopathogenic microorganisms, which comprises the application of a compound of formula I according to claim 1 as active ingredient to the plant, to parts of plants or to the locus thereof.

9. A method according to claim 8, wherein the phytopathogenic microorganisms are fungal organisms.

## Patentansprüche

1. Verbindung der Formel I 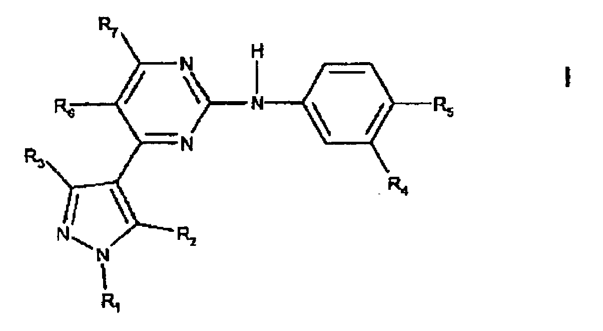 worin
R₁ Wasserstoff, COCH₃, COCH₂CH₃, SO₂N(CH₃)₂, COOCH₃, COOCH₂CH₃, CON(CH₃)₂, SO₂CH₃, CH₂C≡CH, Methyl , Ethyl, n-Propyl oder n-Butyl darstellt;
R₂ Wasserstoff oder Amino darstellt;
R₃ Phenyl oder Thienyl darstellt, wobei jeder davon gegebenenfalls mit einem, zwei oder drei Substituenten R₁₂ substituiert sein kann, wobei jede der Gruppen R₁₂ gleich oder verschieden sein kann;
R₄ Wasserstoff, Hydroxy, Cyano, Fluor, Chlor, Brom, 2-Hydroximino-ethyl, 2-Methoximino-ethyl, C₁-C₄-Alkyl, C₁-C₄₋Alkoxy, C₁-C₄-Halogenalkoxy, C₁-C₄-Halogenalkyl, C₁-C₄-Cyanoalkyl, C₁-C₄-Alkanoyloxy, C₁-C₄-Hydroxyalkyl, C₁-C₄-Halogenalkanoyloxy, C₁-C₄-Alkanoyl-C₁-C₆-aminoalkyl, C₁-C₄-Alkanoyloxy-C₁-C₄-alkyl, C₁-C₄-Alkanoyl, C₁-C₄-Alkylthio, C₁-C₄₋Alkoxycarbonyl, Heteroaryl oder Heterocyclyl, C₁-C₄-Alkanoyloxy, C₁-C₄-Alkyloxycarbonyloxy, C₁-C₄-Alkanoyloxy, Di-(C₁-C₄-alkyl) amino-C₁-C₄-alkanoyloxy oder Di (C₁-C₄-alkyl) - aminosulfonyloxy darstellt;
R₅ Wasserstoff, C₁-C₃-Alkyl, Hydroxy, C₁-C₃-Alkoxy oder Halogen darstellt;
R₆ Wasserstoff, C₁-C₃-Alkyl , Halogen oder C₁-C₃-Alkoxy darstellt;
R₇ Wasserstoff, C₁-C₃-Alkyl, Halogen oder C₁-C₃-Alkoxy darstellt;
R₁₂ Halogen, C₁-C₄-Alkyl oder C₁-C₄-Alkoxy darstellt.

2. Verbindung nach Anspruch 1, die eine Verbindung der Formel IA darstellt: 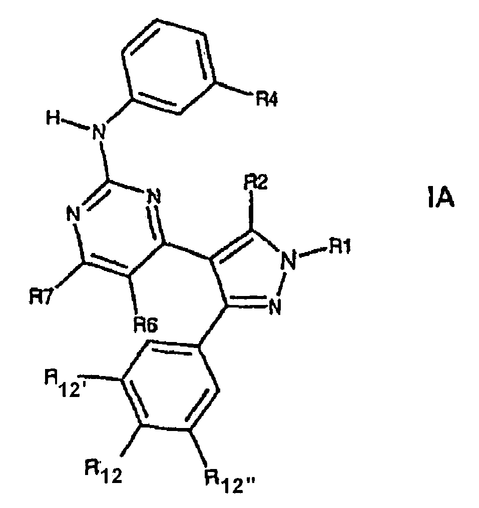 worin R₁, R₂, R₄, R₆ und R₇ wie in Bezug auf Formel I in Anspruch 1 definiert sind und R₁₂, R₁₂' und R₁₂" unabhängig wie für R₁₂ in Formel I definiert sind.

3. Verbindung nach Anspruch 2, worin R₆, R₇, R₁₂' und R₁₂" alle Wasserstoff darstellen.

4. Verbindung nach einem vorangehenden Anspruch, worin R₄ Wasserstoff, Hydroxy, Cyano, Fluor, Chlor, Brom, Methyl, tert-Butyl, Methylthio, Trifluormethyl, Cyanomethyl, 2-Cyanoethyl, Hydroxymethyl, 1-Hydroxyethyl, 2-Hydroxyisopropyl, Acetyl, 2-Hydroximino-ethyl, 2-Methoximino-ethyl, Acetoxymethyl, Methoxycarbonyl, Methoxy, Ethoxy oder Trifluormethoxyoxadiazolyl (gegebenenfalls substituiert mit Methyl oder Trifluormethyl), Piperidinyl, Morpholinyl, Piperazinyl, Pyridyloxy, Pyrimidinyloxy, Thiazolyloxy, Pyridazinyloxy, C₁-C₄-Alkanoyloxy, C₁-C₄-Alkyloxycarbonyloxy, C₁-C₄-Alkanoyloxy, Di (C₁-C₄-alkyl) amino-C₁-C₄-alkanoyloxy oder Di(C₁-C₄-alkyl)aminosulfonyloxy darstellt.

5. Verbindung nach einem vorangehenden Anspruch worin R₁₂ H darstellt oder Fluor, Chlor, Brom, Methyl, Ethyl, Methoxy oder Ethoxy darstellt.

6. Zusammensetzung zum Bekämpfen und Schützen gegen phytopathogene Mikroorganismen, umfassend eine Verbindung der Formel I nach Anspruch 1 als Wirkbestandteil zusammen mit einem geeigneten Träger- oder Verdünnungsmittel.

7. Verwendung einer Verbindung der Formel I nach Anspruch 1 beim Schützen von Pflanzen gegen Befall durch phytopathogene Mikroorganismen.

8. Verfahren zum Bekämpfen und zur Prävention eines Befalls von Kulturpflanzen durch phytopathogene Mikroorganismen, das die Applikation einer Verbindung der Formel I nach Anspruch 1 als Wirkbestandteil auf die Pflanze, Pflanzenteile oder deren Standort umfasst.

9. Verfahren nach Anspruch 8, wobei die phytopathogenen Mikroorganismen Pilzorganismen sind.

## Revendications

1. Composé de formule I 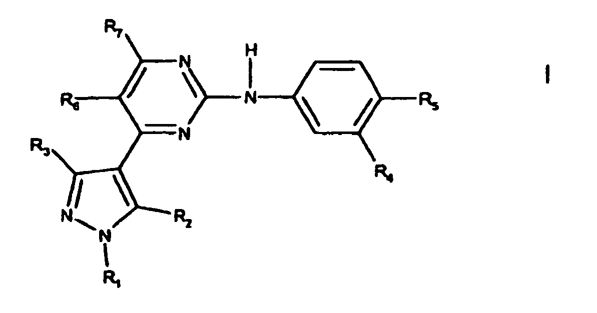 dans laquelle
R₁ est hydrogène, COCH₃, COCH₂CH₃, SO₂N(CH₃)₂, COOCH₃, COOCH₂CH₃, CON(CH₃)₂, SO₂CH₃, CH₂C≡CH, méthyle, éthyle, n-propyle ou n-butyle;
R₂ est hydrogène ou amino;
R₃ est phényle ou thiényle pouvant chacun être éventuellement substitués par 1, 2 ou 3 substituants R₁₂, les groupes R₁₂ pouvant être chacun identiques ou différents,
R₄ est hydrogène, hydroxy, cyano, fluoro, chloro, bromo, 2-hydroxyiminoéthyle, 2-méthoxyiminoéthyle, alkyle en C₁-C₄, alcoxy en C₁-C₄, halogénoalcoxy en C₁-C₄, halogénoalkyle en C₁-C₄, cyanoalkyle en C₁-C₄, alcanoyloxy en C₁-C₄, hydroxyalkyle en C₁-C₄, halogénoalcanoyloxy en C₁-C₄, (alcanoyl en C₁-C₄)-(aminoalkyle en C₁-C₆), (alcanoyloxy en C₁-C₄)-(alkyle en C₁-C₄), alcanoyle en C₁-C₄, alkylthio en C₁-C₄, (alcoxy en C₁-C₄)carbonyle, hétéroaryle ou hétérocyclyle, alcanoyloxy en C₁₋C₄, (alkyl en C₁-C₄)oxycarbonyloxy, alcanoyloxy en C₁-C₄, di(alkyl en C₁₋C₄)amino(alcanoyloxy en C₁-C₄) ou di(alkyl en C₁-C₄)aminosulfonyloxy;
R₅ est hydrogène, alkyle en C₁-C₃, hydroxy, alcoxy en C₁-C₃ ou halogéno;
R₆ est hydrogène, alkyle en C₁-C₃, halogéno ou alcoxy en C₁-C₃;
R₇ est hydrogène, alkyle en C₁-C₃, halogéno ou alcoxy en C₁-C₃;
R₁₂ est halogéno, alkyle en C₁-C₄ ou alcoxy en C₁-C₄.

2. Composé selon la revendication 1, qui est un composé de formule IA 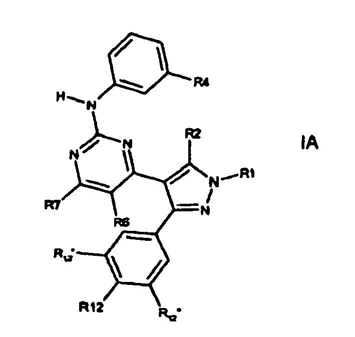 dans laquelle R₁, R₂, R₄, R₆ et R₇ ont la définition donnée en relation avec la formule I dans la revendication 1 et R₁₂, R_{12'} et R_{12''} ont indépendamment la définition donnée pour R₁₂ dans la formule I.

3. Composé selon la revendication 2, dans lequel R₆, R₇, R_{12'} et R_{12''} sont tous l'hydrogène.

4. Composé selon l'une quelconque des revendications précédentes, dans lequel R₄ est hydrogène, hydroxy, cyano, fluoro, chloro, bromo, méthyle, tert-butyle, méthylthio, trifluorométhyle, cyanométhyle, 2-cyanoéthyle, hydroxyméthyle, 1-hydroxyéthyle, 2-hydroxyisopropyle, acétyle, 2-hydroxyiminoéthyle, 2-méthoxyiminoéthyle, acétoxyméthyle, méthoxycarbonyle, méthoxy, éthoxy ou trifluorométhoxy oxadiazolyle (éventuellement substitué par méthyle ou trifluorométhyle), pipéridinyle, morpholinyle, pipérazinyle, pyridyloxy, pyrimidinyloxy, thiazolyloxy, pyridazinyloxy, alcanoyloxy en C₁-C₄, (alkyl en C₁-C₄)oxycarbonyloxy, alcanoyloxy en C₁-C₄, di(alkyl en C₁-C₄)amino(alcanoyloxy en C₁-C₄) ou di(alkyl en C₁₋C₄)aminosulfonyloxy.

5. Composé selon l'une quelconque des revendications précédentes, dans lequel R₁₂ est H ou est fluoro, chloro, bromo, méthyle, éthyle, méthoxy ou éthoxy.

6. Composition destinée à la lutte ou à la protection contre des micro-organismes phytopathogènes, comprenant un composé de formule I selon la revendication 1 en tant qu'ingrédient actif, avec un support ou diluant approprié.

7. Utilisation d'un composé de formule I selon la revendication 1 dans la protection de plantes contre une infestation par des micro-organismes phytopathogènes.

8. Procédé de lutte ou de prévention contre une infestation de plantes cultivées par des micro-organismes phytopathogènes, qui comprend l'application d'un composé de formule I selon la revendication 1 comme ingrédient actif à la plante, à des parties de plantes ou à leur site.

9. Procédé selon la revendication 8, dans lequel les micro-organismes phytopathogènes sont des organismes fongiques.
